# EUROPEAN PATENT SPECIFICATION

(11) **EP 4 344 621 B1**
(45) Date of publication and mention of the grant of the patent: **13.08.2025**
(21) Application number: 22831288.0
(22) Date of filing: 30.03.2022
(51) Int. Cl.: A61B 5/021, A61B 5/0225, A61B 5/024, A61B 5/00

(54) **APPARATUS AND METHOD FOR MEASURING BLOOD PRESSURE**
VORRICHTUNG UND VERFAHREN ZUR BLUTDRUCKMESSUNG
APPAREIL ET PROCÉDÉ POUR MESURER LA PRESSION ARTÉRIELLE

(30) Priority: 30.06.2021 CN 202110747912
(43) Date of publication of application: 03.04.2024
(73) Proprietor: Huawei Technologies Co., Ltd., Longgang Shenzhen, Guangdong 518129 (CN)
(72) Inventor: FU, Xiaoyu, Shenzhen, Guangdong 518129 (CN); HUANG, Zhenlong, Shenzhen, Guangdong 518129 (CN)
(74) Representative: Epping - Hermann - Fischer
(86) International application number: PCT/CN2022/084070
(87) International publication number: WO 2023/273457

(56) References cited:
- EP-A1- 3 572 980
- EP-A1- 3 572 980
- CN-A- 106 821 356
- CN-A- 106 821 356
- CN-A- 110 731 762
- CN-A- 110 731 762
- CN-A- 110 840 427
- CN-A- 110 840 427
- US-A1- 2021 193 311
- US-A1- 2021 193 311
- US-B2- 10 045 700

## Description

This disclosure claims priority to Chinese Patent Application No. 202110747912.5, filed with the China National Intellectual Property Administration on June 30, 2021 and entitled "BLOOD PRESSURE MEASUREMENT APPARATUS AND METHOD".

### TECHNICAL FIELD

This disclosure relates to the field of terminal technologies, and more specifically, to a blood pressure measurement apparatus and method.

### BACKGROUND

At present, a quantity of hypertension patients in China accounts for a very large proportion of a total quantity of cardiovascular patients. For the hypertension patients, it is essential to measure blood pressure for daily monitoring of the blood pressure.

Blood pressure measurement methods usually include an auscultation method and an oscilloscope method. In both the methods, a cuff needs to be manually or automatically inflated and deflated after the cuff is wrapped around an arm of a person. Such a blood pressure measurement method may be referred to as a cuff-based measurement method, and a corresponding device may be referred to as a cuff-based blood pressure measurement device.

Although measurement accuracy of the cuff-based blood pressure measurement device can reach a medical standard, the device is inconvenient to operate and easily causes discomfort to a user. Therefore, it is necessary to provide a blood pressure measurement device that is easily operated and that has high measurement accuracy.

Document US 2021/193311 A1 describes a system for continuous non-invasive blood pressure monitoring which includes processing circuitry configured to: determine calibration data for a continuous non-invasive blood pressure model at a calibration point, receive, from an oxygen saturation sensing device, a PPG signal at a particular time subsequent to the calibration point, derive values of the set of metrics for the patient from the PPG signal, and determine, using the continuous non-invasive blood pressure model and based at least in part on inputting the calibration data determined at the calibration point, the values of the set of metrics, and an elapsed time at the particular time since the calibration point into the continuous non-invasive blood pressure model, a blood pressure of the patient at the particular time.

Document CN 110 840 427 A provides a continuous blood pressure measuring method and device based on a volume pulse wave signal, equipment and a storage medium. The method includes: acquiring the volume pulse wave signal of a to-be-measured object, wherein the volume pulse wave signal at least comprises a pulse wave period; acquiring a blood pressure feature value according to the periodic value of each pulse vale and the amplitude change value of the pulse wave from the starting point of each pulse wave period to preset time; inputting the blood pressure feature value into a pre-trained linear blood pressure model of the to-be-measured object to obtain the blood pressure value of the to-be-measured object. According to this document, such a method has the advantages that a cuff type blood measuring method which needs to wait for cuff inflation and deflation during blood pressure measuring is avoided, practical application is facilitated, and continuous blood pressure measuring is achieved; the blood pressure feature value uses the amplitude information and speed information of the volume pulse wave signal at the same time, blood pressure measuring accuracy is increased, the linear blood pressure model is simple in calculation, and blood pressure measuring efficiency is increased.

Document CN 106 821 356 A discloses a cloud continuous blood pressure measurement method and system based on an Elman neural network. The method comprises the following steps: obtaining real-time pulse wave signals of a subject through measurement; performing denoising processing on the pulse wave signals; extracting feature points from the denoised pulse wave signals; S4, taking the extracted feature points of the pulse wave signals as input of the Elman neural network, predicting blood pressure value with a well-trained Elman neural network model, and taking obtained prediction values as continuous blood pressure measured values. Based on the Elman neural network, the method can predict the blood pressure values accurately, has better accuracy and stability and can be widely applied to the industry of blood pressure measurement. Document US 10 045 700 B2 describes a blood pressure estimating method which includes measuring a biosignal including pulse wave information of a user, determining a calibration method for a blood pressure estimation model, calibrating the blood pressure estimation model using the determined calibration method, and estimating a blood pressure of the user from the biosignal using the calibrated blood pressure estimation model.

Document CN 110 731 762 A relates to a method and apparatus for preprocessing a pulse wave based on a similarity. This document further describes a computer system and a readable storage medium which relate to the field of medical digital signal processing. The method comprises the following steps: collecting a pulse wave, and partitioning, based on a rhythm, the pulse wave into a plurality of periodic waveforms; calculating a similarity between any two periodic waveforms to obtain similarity information corresponding to all the periodic waveforms; screening out a template wave according to the similarity information of all the periodic waveforms; and preprocessing, based on the template wave, the pulse wave. Abnormal waveforms are screened and abandoned by means of similarity comparison, thereby achieving the aim of preprocessing the pulse wave, improving the accuracy of a subsequent feature extraction result of the waveforms, and facilitating application of subsequent waveform processing.

Document EP 3 572 980 A1 describes a method and a system for a feature subset-classifier pair for a classification task.

### SUMMARY

This disclosure provides a blood pressure measurement apparatus and method. The apparatus can improve accuracy of a measurement result when measuring a blood pressure value of a user, and can be easily operated.

The present invention is set out by the set of appended claims. In the following, parts of the description and drawing referring to implementations or examples, which are not covered by the claims are not presented as embodiments of the invention, but as illustrative examples useful for understanding the invention. In particular, the embodiments of the invention are defined by the appended claims.

According to a first embodiment, a blood pressure measurement apparatus is provided. The blood pressure measurement apparatus includes a processor 101, a first measurement component 102, and a second measurement component 103. The first measurement component 102 includes an inflatable part 1022, an airbag 1023, and an air pressure sensor 1024. The airbag 1023 is separately connected to the inflatable part 1022 and the air pressure sensor 1024. The second measurement component 103 includes a light source 1031 and a PPG sensor 1032. The first measurement component 102 is configured to collect a blood pressure value of a user. The second measurement component 103 is configured to collect a PPG signal of the user. The processor 101 is configured to: control the first measurement component 102 to collect N sets of blood pressure values of the user; control the second measurement component 103 to collect N sets of PPG signals corresponding to the N sets of blood pressure values; and generate a target model based on the N sets of blood pressure values and the N sets of PPG signals. An input of the target model is the PPG signal, and an output of the target model is the blood pressure value. N is an integer greater than or equal to 2. The processor 101 is further configured to: after generating the target model, control the second measurement component 103 to collect a first PPG signal of the user; and determine a first blood pressure value based on the target model and the first PPG signal.

According to the foregoing technical solution, the target model is obtained by model training based on the N sets of blood pressure values of the user (where the N sets of blood pressure values are obtained through the first measurement component 102, and accuracy of the blood pressure values obtained through the first measurement component 102 is high) and the N sets of PPG signals in one-to-one correspondence with the N sets of blood pressure values. Therefore, when the blood pressure value of the user is subsequently determined by a target model 2 based on the PPG signal of the user, an obtained measurement result of the blood pressure value is more accurate.

In addition, the second measurement component configured to collect the PPG signal is integrated with the first measurement component, so that the N sets of blood pressure values and the N sets of PPG signals in one-to-one correspondence with the N sets of blood pressure values that are used to generate the target model can be obtained more conveniently. The target model in this disclosure corresponds to the foregoing target model 2.

The processor is further configured to: when the i^{th} set of PPG signals in the N sets of PPG signals is collected, control the second measurement component 103 to obtain a PPG signal of a first duration; determine a category of the PPG signal of the first duration; determine a similarity between the PPG signal of the first duration and a center of the category of the PPG signal of the first duration in first historical PPG signals, where the first historical PPG signals include a PPG signal that has been obtained before the i^{th} set of PPG signals is obtained; determine a second duration based on the similarity; control the second measurement component 103 to collect a PPG signal of the second duration; and combine the PPG signal of the first duration and the PPG signal of the second duration into the i^{th} set of PPG signals.

According to the foregoing technical solution, the PPG signal of the first duration is first obtained, the category of the PPG signal of the first duration is determined, the similarity between the PPG signal of the first duration and the center of the category of the PPG signal of the first duration in the first historical PPG signals is further determined, and whether to continuously collect a PPG signal of the second duration is determined based on the similarity. In this way, a collection duration of a set of PPG signals can be flexibly determined without affecting measurement accuracy of the target model 2.

In a possible implementation, the processor is further configured to: when 1^{st} to M^{th} sets of PPG signals in the N sets of PPG signals are collected, control the second measurement component 103 to obtain a PPG signal of a third duration, where M is an integer less than i.

In a possible implementation, the processor 101 is further configured to: when determining to update the target model, prompt the user to trigger an instruction for measuring blood pressure through the first measurement component 102 and the second measurement component 103; in response to a first operation of the user, control the first measurement component 102 to collect a second blood pressure value, and control the second measurement component 103 to collect a second PPG signal corresponding to the second blood pressure value; and update the target model based on the second blood pressure value and the second PPG signal.

According to the foregoing technical solution, to further improve measurement accuracy of the target model 2, when it is determined that the target model 2 needs to be updated, the user is prompted to trigger the instruction for measuring blood pressure through the first measurement component. Then, the target model 2 is updated based on the blood pressure value and the PPG signal that are obtained based on the instruction, triggered by the user, for measuring blood pressure through the first measurement component.

In a possible implementation, the processor 101 is further configured to determine a category of the first PPG signal; and
the processor 101 is further configured to: determine a similarity between the first PPG signal and a center of PPG signals of the category of the first PPG signal in second historical PPG signals, and determine, based on the similarity and a quantity of sets of PPG signals of the category of the first PPG signal in the second historical PPG signals, whether the target model needs to be updated, where the second historical PPG signals include the N sets of PPG signals.

In a possible implementation, the processor 101 is further configured to determine, based on a duration in which the target model is not updated, whether the target model needs to be updated. In a possible implementation, the inflatable part 1022 is configured to inflate the airbag 1023; and
the air pressure sensor 1024 is configured to collect a plurality of air pressure values of the airbag 1023, where a first air pressure value and a second air pressure value in the plurality of air pressure values are blood pressure values of the user, the first air pressure value is an air pressure value corresponding to a moment at which an oscillatory wave of air pressure in the airbag 1023 reaches a maximum value, the second air pressure value is an air pressure value corresponding to a moment at which the oscillatory wave reaches the maximum value multiplied by a, and a is greater than 0 and less than 1.

In a possible implementation, a difference between a moment at which the airbag 1023 starts to be inflated and a collection moment of the i^{th} set of PPG signals is greater than or equal to a preset first threshold and is less than or equal to a preset second threshold, and the moment at which the airbag 1023 starts to be inflated is after the collection moment of the i^{th} set of PPG signals, or a difference between a collection moment of the i^{th} set of PPG signals and a moment at which inflation of the airbag 1023 is stopped is greater than or equal to a preset third threshold and is less than or equal to a preset fourth threshold, and the moment at which inflation of the airbag 1023 is stopped is before the collection moment of the i^{th} set of PPG signals.

When an i^{th} set of blood pressure values of the user is collected, the inflatable part 1022 needs to inflate the airbag 1023. Because an inflated airbag 1023 exerts pressure on a wrist part of the user, accuracy of the obtained i^{th} set of PPG signals of the user is affected.

To avoid impact on the accuracy of the PPG signal, the collection moment of the i^{th} set of PPG signals is set as a moment before the airbag 1023 starts to be inflated or a moment after inflation of the airbag 1023 is stopped, so that a time period in which the i^{th} set of PPG signals is collected does not overlap a time period in which gas inside the airbag 1023 exerts pressure on the wrist part of the user. In other words, the i^{th} set of PPG signals is collected in a time period other than the time period in which the gas inside the airbag 1023 exerts pressure on the wrist part of the user. In this way, the accuracy of the obtained i^{th} set of PPG signals of the user is improved.

According to a second embodiment, a blood pressure measurement method is provided. The method is applied to a blood pressure measurement apparatus. The blood pressure measurement apparatus includes a first measurement component 102 and a second measurement component 103. The first measurement component 102 includes an inflatable part 1022, an airbag 1023, and an air pressure sensor 1024. The airbag 1023 is separately connected to the inflatable part 1022 and the air pressure sensor 1024. The second measurement component 103 includes a light source 1031 and a PPG sensor 1032. The method includes: controlling the first measurement component 102 to collect N sets of blood pressure values of a user; controlling the second measurement component 103 to collect N sets of PPG signals corresponding to the N sets of blood pressure values; and generating a target model based on the N sets of blood pressure values and the N sets of PPG signals, where an input of the target model is the PPG signal, an output of the target model is the blood pressure value, and N is an integer greater than or equal to 2; after generating the target model, controlling the second measurement component to collect a first PPG signal of the user; and determining a first blood pressure value based on the target model and the first PPG signal. When an i^{th} set of PPG signals in the N sets of PPG signals is collected, the controlling the second measurement component 103 to collect N sets of PPG signals corresponding to the N sets of blood pressure values includes: controlling the second measurement component 103 to obtain a PPG signal of a first duration; determining a category of the PPG signal of the first duration; determining a similarity between the PPG signal of the first duration and a center of the category of the PPG signal of the first duration in first historical PPG signals, where the first historical PPG signals include a PPG signal that has been obtained before the i^{th} set of PPG signals is obtained; determining a second duration based on the similarity; controlling the second measurement component 103 to collect a PPG signal of the second duration; and combining the PPG signal of the first duration and the PPG signal of the second duration into the i^{th} set of PPG signals.

In a possible implementation, when 1^{st} to M^{th} sets of PPG signals in the N sets of PPG signals are collected, the controlling the second measurement component 103 to collect N sets of PPG signals corresponding to the N sets of blood pressure values includes: controlling the second measurement component 103 to obtain a PPG signal of a third duration, where M is an integer less than i.

In a possible implementation, the method further includes: when determining to update the target model, prompting the user to trigger an instruction for measuring blood pressure through the first measurement component 102 and the second measurement component 103; in response to a first operation of the user, controlling the first measurement component 102 to collect a second blood pressure value, and controlling the second measurement component 103 to collect a second PPG signal corresponding to the second blood pressure value; and updating the target model based on the second blood pressure value and the second PPG signal.

In a possible implementation, the method further includes: determining a category of the first PPG signal; and
determining a similarity between the first PPG signal and a center of PPG signals of the category of the first PPG signal in second historical PPG signals, and determining, based on the similarity and a quantity of sets of PPG signals of the category of the first PPG signal in the second historical PPG signals, whether the target model needs to be updated, where the second historical PPG signals include the N sets of PPG signals.

In a possible implementation, the method further includes: determining, based on a duration in which the target model is not updated, whether the target model needs to be updated.

In a possible implementation, the controlling the first measurement component 102 to collect N sets of blood pressure values of a user includes: controlling the inflatable part 1022 to inflate the airbag 1023; and controlling the air pressure sensor 1024 to collect a plurality of air pressure values of the airbag 1023, where a first air pressure value and a second air pressure value in the plurality of air pressure values are a set of blood pressure values of the user, the first air pressure value is an air pressure value corresponding to a moment at which an oscillatory wave of air pressure in the airbag 1023 reaches a maximum value, the second air pressure value is an air pressure value corresponding to a moment at which the oscillatory wave reaches the maximum value multiplied by a, and a is greater than 0 and less than 1.

In a possible implementation, a difference between a moment at which the airbag 1023 starts to be inflated and a collection moment of the i^{th} set of PPG signals is greater than or equal to a preset first threshold and is less than or equal to a preset second threshold, and the moment at which the airbag 1023 starts to be inflated is after the collection moment of the i^{th} set of PPG signals, or a difference between a collection moment of the i^{th} set of PPG signals and a moment at which inflation of the airbag 1023 is stopped is greater than or equal to a preset third threshold and is less than or equal to a preset fourth threshold, and the moment at which inflation of the airbag 1023 is stopped is before the collection moment of the i^{th} set of PPG signals.

For beneficial effects of any implementation of the second embodiment, refer to related descriptions of the first embodiment. For brevity, details are not described herein again.

According to a third embodiment, a blood pressure measurement apparatus is provided. The apparatus has a function of implementing behavior of the blood pressure measurement apparatus in any method in the foregoing embodiments and possible designs. This function may be implemented by hardware, or may be implemented by executing corresponding software by hardware. The hardware or the software includes at least one module or unit corresponding to the foregoing function, for example, a measurement module or unit, a sensing module or unit, or an inflatable module or unit.

According to a fourth embodiment, a computer storage medium is provided. The computer storage medium includes computer instructions. When the computer instructions are run on a blood pressure measurement apparatus, the blood pressure measurement apparatus is enabled to perform the blood pressure measurement method in any possible design of the foregoing embodiments. According to a fifth embodiment, a computer program product is provided. When the computer program product runs on a computer, the computer is enabled to perform the blood pressure measurement method in any possible design of the foregoing embodiments.

### BRIEF DESCRIPTION OF DRAWINGS

FIG. 1 is a schematic block diagram of a blood pressure measurement apparatus 100 according to this disclosure;
FIG. 2 is a schematic diagram of electronic control logic of a blood pressure measurement apparatus 100 according to this disclosure;
FIG. 3 is a schematic diagram of a structure of a blood pressure measurement apparatus 100 according to this disclosure;
FIG. 4 is a schematic diagram of a distribution status of a plurality of light sources and a plurality of photoelectric sensors on an apparatus body according to this disclosure;
FIG. 5 is a schematic diagram of a watch for measuring blood pressure that is worn by a user according to this disclosure;
FIG. 6 is a schematic diagram of a display interface of a display screen of a watch for measuring blood pressure according to this disclosure; and
FIG. 7A and FIG. 7B are a schematic flowchart of a blood pressure measurement method according to this disclosure.

### DETAILED DESCRIPTION

The following describes technical solutions in examples of this disclosure with reference to the accompanying drawings. Unless otherwise specified, "/" in descriptions of this disclosure represents an "or" relationship between associated objects. For example, A/B may represent A or B. In this disclosure, "and/or" is merely an association relationship for describing associated objects, and represents that three relationships may exist. For example, A and/or B may represent the following three cases: Only A exists, both A and B exist, and only B exists, where A and B may be singular or plural. In addition, in the descriptions of this disclosure, unless otherwise specified, "a plurality of" refers to two or more. "At least one of the following items (pieces)" or similar expressions refer to any combination of these items, including any combination of singular items (pieces) or plural items (pieces). For example, at least one item (piece) of a, b, or c may represent: a, b, c, a and b, a and c, b and c, or a, b, and c, where a, b, and c may be singular or plural. In addition, to clearly describe the technical solutions in examples of this disclosure, in examples of this disclosure, terms such as "first" and "second" are used to distinguish between same objects or similar objects whose functions and purposes are basically the same. A person skilled in the art may understand that the terms such as "first" and "second" do not limit a quantity or an execution sequence, and the terms such as "first" and "second" do not limit a definite difference.

This disclosure provides a blood pressure measurement apparatus 100.

In an implementation, a target model 1 may be pre-configured in the apparatus 100. The target model 1 represents a correspondence between a PPG signal and a blood pressure value. For example, the target model 1 may be pre-configured for the apparatus 100 before delivery of the apparatus 100. An input of the target model 1 may be a photoplethysmogram (photoplethysmogram, PPG) signal of a user, and an output of the target model may be a set of blood pressure values of the user. In other words, after a PPG signal of the user is input into the target model 1, a set of blood pressure values of the user may be obtained through the target model 1.

It should be understood that a set of blood pressure values of the user in this disclosure includes systolic blood pressure (systolic blood pressure, SBP) and diastolic blood pressure (diastolic blood pressure, DBP) of the user. For ease of description, in this disclosure, a set of blood pressure values is used to replace the systolic blood pressure and the diastolic blood pressure of the user.

To improve accuracy of the blood pressure value of the user obtained based on the PPG signal of the user, a plurality of sets of PPG signals of the user and a plurality of sets of blood pressure values of the user can be used (where the plurality of sets of blood pressure values of the user are not obtained through the target model 1, but are obtained through a first measurement component in the following) to update the target model 1 to obtain a target model 2. The plurality of sets of blood pressure values are in one-to-one correspondence with the plurality of sets of PPG signals. The target model 2 is obtained by updating the target model 1 based on the plurality of sets of blood pressure values and the plurality of sets of PPG signals of the user. Therefore, a blood pressure value of the user that is obtained through the target model 2 and based on the PPG signal of the user is more accurate than that obtained through the target model 1. The target model 2 in this disclosure corresponds to a target model.

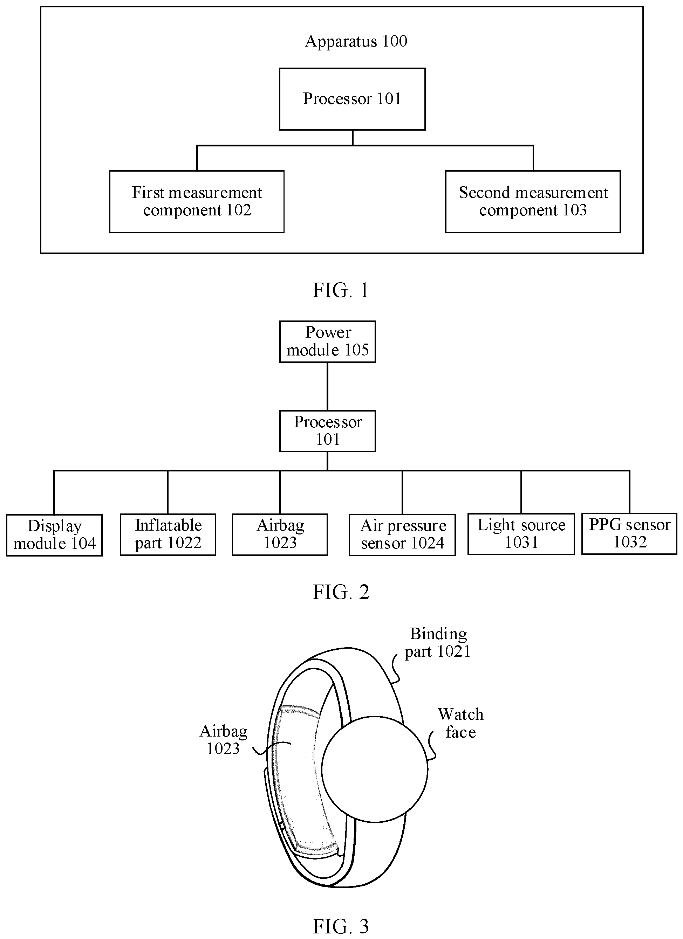

In this case, the apparatus 100 includes a processor 101, a first measurement component 102, and a second measurement component 103. FIG. 1 is a schematic block diagram of the apparatus 100. For example, the first measurement component 102 is configured to collect a blood pressure value of the user. In other words, the first measurement component 102 collects blood pressure of the user for N times, to obtain N sets of blood pressure values of the user, where N is an integer greater than 2. For example, after detecting an instruction, triggered by the user, for measuring blood pressure through the first measurement component 102, the processor 101 may send an instruction for collecting blood pressure to the first measurement component 102, and the first measurement component 102 executes the instruction for collecting blood pressure to collect the blood pressure value of the user.

The second measurement component 103 is configured to collect a PPG signal of the user. The processor 101 is configured to control the second measurement component 103 to collect a set of PPG signals corresponding to the blood pressure value of the user. For a method for obtaining the set of PPG signals corresponding to the blood pressure value of the user by the processor 101, refer to related descriptions in the following.

It should be noted that the blood pressure value of the user and the set of PPG signals corresponding to the blood pressure value in this disclosure are obtained by measuring a same part of the user.

The processor 101 is configured to update the target model 1 based on the N sets of blood pressure values and N sets of PPG signals of the user, to obtain the target model 2. The target model 2 represents a correspondence between the PPG signal and the blood pressure value of the user. An input of the target model 2 is the PPG signal of the user, and an output of the target model is the blood pressure value of the user.

When the blood pressure of the user is measured based on the target model 2, the processor 101 is further configured to: after obtaining a first PPG signal of the user, input the first PPG signal into the target model 2, to obtain a first blood pressure value of the user.

It should be noted that, that the target model 1 is pre-configured in the foregoing processor 101 is merely an example, and constitutes no limitation on this disclosure. During specific implementation, the target model 1 may not be pre-configured in the processor 101, but the processor 101 directly generates the target model 2 based on the N sets of blood pressure values and the N sets of PPG signals.

Optionally, the first measurement component 102 may include an inflatable part 1022, an airbag 1023, and an air pressure sensor 1024. The airbag 1023 is separately connected to the inflatable part 1022 and the air pressure sensor 1024.

When the blood pressure value of the user is collected, the inflatable part 1022 is configured to inflate the airbag 1023; and the air pressure sensor 1024 is configured to collect a plurality of air pressure values of the airbag 1023. In the plurality of air pressure values, an air pressure value corresponding to a moment at which an oscillatory wave of air pressure in the airbag 1023 reaches a maximum value is a first air pressure value, an air pressure value corresponding to a moment at which the oscillatory wave of the air pressure in the airbag 1023 reaches the maximum value multiplied by a is a second air pressure value, the first air pressure value is systolic blood pressure of the user, the second air pressure value is diastolic blood pressure of the user, and a is greater than 0 and less than 1.

For example, if the air pressure value corresponding to the moment at which the oscillatory wave of the air pressure in the airbag 1023 reaches the maximum value is 128, 128 may be determined as the systolic blood pressure of the user. Assuming that a value of a is 0.45 and that an air pressure value corresponding to a moment at which the oscillatory wave of the air pressure in the airbag 1023 reaches the maximum value multiplied by 0.45 is 80, 80 may be determined as the diastolic blood pressure of the user. In this case, the systolic blood pressure of 128 and the diastolic blood pressure of 80 form a set of blood pressure values of the user.

Optionally, the second measurement component 103 may include a plurality of light sources 1031 and a plurality of PPG sensors 1032. The plurality of light sources 1031 and the plurality of PPG sensors 1032 may be disposed on a contact surface between the apparatus 100 and a body part of the user.

When the PPG signal is collected, the plurality of light sources 1031 are configured to emit light, and the plurality of PPG sensors 1032 are configured to obtain reflected light obtained by reflecting the emitted light by the body part that is of the user and that is in contact with the apparatus 100, and convert a received optical signal into a PPG signal.

Optionally, the apparatus 100 may further include a display module 104 and a power module 105. The power module 105 is configured to supply power to the apparatus 100. The display module 104 is configured to display the blood pressure value of the user to the user. FIG. 2 is a schematic diagram of electronic control logic of the apparatus 100. The processor 101 may be a microcontroller unit (microcontroller unit, MCU), and the display module 104 may be a display screen.

Optionally, the apparatus 100 is a watch for measuring blood pressure. FIG. 3 is a side view of the watch. In this case, the apparatus 100 may further include a binding part 1021. The binding part 1021 may be a watchband of the watch. The airbag 1023 may be bonded to the inside of the watchband, or may be fastened in the watchband through a buckle. The display module 104 may be a display screen of the watch. It should be noted that when the apparatus 100 is the watch, the air pressure sensor 1024 (not shown in FIG. 3) and the inflatable part 1022 (not shown in FIG. 3) are deployed inside a watch face.

When the apparatus 100 is the watch, the foregoing plurality of light sources 1031 and the foregoing plurality of PPG sensors 1032 may be distributed on a back surface of the watch face.

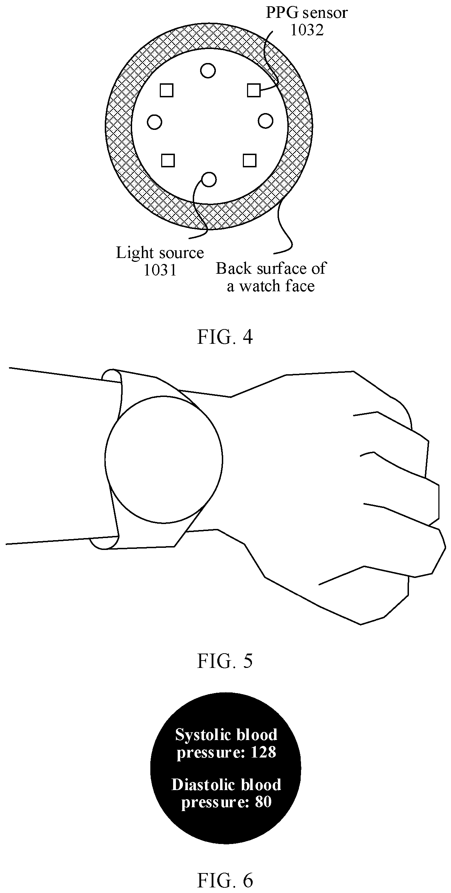

FIG. 4 is a schematic diagram of a distribution status of the plurality of light sources 1031 and the plurality of PPG sensors 1032 on the back surface of the watch face. For example, the light source 1031 may be a light-emitting diode (light-emitting diode, LED) that emits green light.

After the user wears the watch on a wrist part, if detecting an instruction, triggered by the user, for measuring blood pressure through the first measurement component 102, the processor 101 may start to collect a blood pressure value of the user, and the processor 101 may send, to the inflatable part 1022, an instruction for instructing to inflate the airbag 1023. The inflatable part 1022 executes the instruction to inflate the airbag 1023. In addition, the processor 101 may send, to the air pressure sensor 1024, an instruction for collecting an air pressure value of the airbag 1023. The air pressure sensor 1024 executes the instruction, to collect a plurality of air pressure values of the airbag 1023. The processor 101 determines a blood pressure value of the user based on a first air pressure value and a second air pressure value in the plurality of air pressure values. FIG. 5 is a schematic diagram of a watch for measuring blood pressure that is worn by a user.

When the PPG signal of the user is collected, the light source 1031 first emits light. After emitted light is reflected by a wrist of the user, the PPG sensor 1032 obtains reflected light obtained by reflecting the emitted light by the wrist of the user, and converts an obtained optical signal into a PPG signal.

After the first blood pressure value of the user is obtained based on the first PPG signal and the target model 2 of the user, the processor 101 may display the first blood pressure value to the user through the display screen of the watch. In this case, a display interface of the display screen of the watch may be shown in FIG. 6.

In addition, the processor 101 may further display, to the user through the display screen of the watch, the blood pressure value of the user that is obtained through the first measurement component 102.

It should be noted that, after determining the blood pressure value, the processor 101 may display the blood pressure value to the user, or may output the blood pressure value by using a voice. This is not limited in this disclosure.

Optionally, a collection moment of the PPG signal of the user may be a moment before the airbag 1023 starts to be inflated or a moment after inflation of the airbag 1023 is stopped. In other words, a difference between a moment at which the airbag 1023 starts to be inflated and the collection moment of the PPG signal of the user is greater than or equal to a preset first threshold and is less than or equal to a preset second threshold, or a difference between the collection moment of the PPG signal of the user and a moment at which inflation of the airbag 1023 is stopped is greater than or equal to a preset third threshold and is less than or equal to a preset fourth threshold. When the blood pressure value of the user is collected, the inflatable part 1022 needs to inflate the airbag 1023. Because an inflated airbag 1023 exerts pressure on the wrist part of the user, accuracy of the obtained PPG signal of the user is affected. Therefore, to avoid impact on accuracy of the PPG signal, a time period in which the PPG signal of the user is collected may not overlap a time period in which gas inside the airbag 1023 exerts pressure on the wrist part of the user. In other words, an i^{th} set of PPG signals may be collected in a time period other than the time period in which the gas inside the airbag 1023 exerts pressure on the wrist part of the user.

For example, the processor 101 may determine a moment before the airbag 1023 starts to be inflated or a moment after inflation of the airbag 1023 is stopped as a collection moment of the i^{th} set of PPG signals, so that the time period in which the PPG signal of the user is collected does not overlap the time period in which the gas inside the airbag 1023 exerts pressure on the wrist part of the user.

In some examples, to keep physical statuses of the user consistent as much as possible between the moment at which the PPG signal is collected and a moment at which the blood pressure value is measured, the foregoing two moments may be close. For example, the preset first threshold is 10 seconds, and the preset second threshold is 40 seconds. The processor 101 starts to inflate the airbag 1023 at a moment t₁, and a moment corresponding to t₁ - (30 seconds) may be used as the collection moment of the i^{th} set of PPG signals. Alternatively, the preset third threshold is 5 seconds, and the preset fourth threshold is 20 seconds. The processor 101 stops inflating the airbag 1023 at a moment t₂, and a moment corresponding to t₂ + (10 seconds) may be used as the collection moment of the i^{th} set of PPG signals. Therefore, during collection of the i^{th} set of PPG signals, the gas inside the airbag 1023 does not exert pressure on the wrist part of the user.

Optionally, when the foregoing i^{th} set of PPG signals in the N sets of PPG signals is collected, the second measurement component 103 is configured to obtain a PPG signal of a first duration. The processor 101 is configured to: determine a category of the PPG signal of the first duration, where for ease of description, the category of the PPG signal of the first duration is denoted as a first category; further determine a similarity between the PPG signal of the first duration and a center of a plurality of sets of PPG signals of the first category in first historical PPG signals; and send, to the second measurement component 103 based on the similarity, an instruction for collecting a PPG signal of a second duration. The second measurement component 103 is configured to collect the PPG signal of the second duration when receiving the instruction for collecting the PPG signal of the second duration. In this case, the i^{th} set of PPG signals includes the PPG signal of the first duration and the PPG signal of the second duration. A value of the foregoing second duration may be related to the similarity.

It is assumed that when the target model 1 is updated, the N sets of blood pressure values of the user and the N sets of PPG signals of the user are collected in total. When the N sets of PPG signals of the user are collected, the processor 101 may first collect several sets of PPG signals based on a fixed duration and classify the several sets of PPG signals. When PPG signals other than the several sets in the N sets are subsequently collected, for each set of PPG signals, the processor 101 may first collect a PPG signal of a specific duration, determine a category of the PPG signal of the duration, further determine a similarity between the PPG signal of the duration and a center of a plurality of sets of PPG signals of the category in the foregoing several sets of PPG signals, and determine, based on the similarity, whether a PPG signal of the duration needs to be continuously collected. When it is determined that the PPG signal does not need to be continuously collected, the PPG signal of the duration that is first collected is used as a set of PPG signals. When it is determined that the PPG signal needs to be continuously collected, the PPG signal that is continuously collected and the PPG signal of the duration that is first collected form a set of PPG signals. According to the method, a collection duration of a set of PPG signals can be flexibly determined. The fixed duration in this disclosure corresponds to a third duration.

It is assumed that when the target model 1 is updated, 50 sets of blood pressure values of the user and 50 sets of PPG signals of the user are collected in total. For the 50 sets of PPG signals of the user, it is assumed that the processor 101 first collects the first 30 sets of PPG signals based on the fixed duration, that is, a value of M is 30. PPG signals other than the first 30 sets of PPG signals in the foregoing 50 sets of PPG signals of the user may be collected in a manner of dynamically determining a collection duration. The following first describes a collection process of the first 30 sets of PPG signals by using an example.

For example, the processor 101 first collects 30 sets of PPG signals based on the fixed duration, and then classifies the 30 sets of PPG signals based on a k-means clustering algorithm (k-means clustering algorithm). The first 30 sets of PPG signals herein correspond to the first historical PPG signals.

For example, features of each of the foregoing 30 sets of PPG signals are extracted. For example, the features may include features such as a pulse head wave peak, a dicrotic wave height, and a duration of a rising period of a waveform corresponding to the PPG signals. Based on the extracted features, the 30 sets of PPG signals are classified based on the k-means clustering algorithm. For example, the first 30 sets of PPG signals are of three categories that are denoted as a category 1, a category 2, and a category 3 for ease of description.

The last 20 sets of PPG signals in the 50 sets of PPG signals of the user may be collected in the following manner. The following describes a collection process of the last 20 sets of PPG signals by using the 31^{st} set of PPG signals as an example.

For the 31^{st} set of PPG signals, the processor 101 may first obtain PPG signals corresponding to three pulse cycles of the user. The processor 101 may determine, based on a k-nearest neighbor classification (k-nearest neighbor classification) algorithm, a most possible category of the PPG signals corresponding to the foregoing three pulse cycles. For example, the processor 101 determines, based on the k-nearest neighbor classification algorithm, that the PPG signals corresponding to the three pulse cycles are most likely of the category 2 in the foregoing three categories. The three pulse cycles herein correspond to the first duration. The following describes a method for determining, based on the k-nearest neighbor classification algorithm, the most possible category of the PPG signals corresponding to the foregoing three pulse cycles.

After determining the most possible category of the PPG signals corresponding to the three pulse cycles, the processor 101 may determine a similarity between the foregoing feature of the PPG signals corresponding to the three pulse cycles and a corresponding feature of a center PPG signal of a plurality of sets of PPG signals of the category 2 in the first 30 sets of PPG signals. The following describes a method for determining the center PPG signal of the plurality of sets of PPG signals of the category 2.

For example, it is assumed that the PPG signals of the category 2 include: the 2^{nd} set, the 6^{th} set, the 7^{th} set, the 11^{th} set, the 14^{th} set, the 17^{th} set, and the 18^{th} set of PPG signals. It is assumed that a center of the 2^{nd} set, the 6^{th} set, the 7^{th} set, the 11^{th} set, the 14^{th} set, the 17^{th} set, and the 18^{th} set of PPG signals of the category 2 is the 6^{th} set of PPG signals. The processor 101 may determine a normalized distance between pulse head wave peaks, dicrotic wave heights, and durations of rising periods of the PPG signals corresponding to the three pulse cycles and a pulse head wave peak, a dicrotic wave height, and a duration of a rising period of the 6^{th} set of PPG signals. The normalized distance corresponds to the similarity in this disclosure, in other words, the normalized distance can represent the similarity. For example, a smaller value of the normalized distance indicates a higher similarity. Conversely, a smaller value of the normalized distance indicates a lower similarity. It should be noted that the 6^{th} set of PPG signals herein corresponds to the center of the plurality of sets of PPG signals of the first category in the first historical PPG signals.

The processor 101 may determine, based on the normalized distance, whether to continuously obtain a PPG signal. When the PPG signal needs to be continuously obtained, the processor 101 may continuously obtain a PPG signal of the second duration.

For example, the processor 101 may compare the normalized distance between the PPG signals corresponding to the foregoing three pulse cycles and the 6^{th} set of PPG signals with a preset fifth threshold. When the normalized distance is less than or equal to the preset fifth threshold, the processor 101 may determine that the PPG signal does not need to be continuously obtained. In this case, the PPG signals corresponding to the foregoing three pulse cycles are the 31^{st} set of PPG signals. Alternatively, when the normalized distance is greater than the preset fifth threshold, the processor 101 may determine that the PPG signal needs to be continuously obtained, and further continuously obtain the PPG signal of the second duration. In this case, the PPG signal of the second duration that is continuously obtained and the PPG signals corresponding to the foregoing three pulse cycles j ointly form the 31^{st} set of PPG signals. For example, if the preset fifth threshold is 0.3, and the normalized distance between the PPG signals corresponding to the foregoing three pulses cycles and the 6^{th} set of PPG signals is 0.2, the processor 101 may determine that the PPG signal does not need to be continuously obtained. Alternatively, if the normalized distance between the PPG signals corresponding to the foregoing three pulses cycles and the 6^{th} set of PPG signals is 0.5, the processor 101 may determine that the PPG signal needs to be continuously obtained, and then continuously obtain the PPG signal of the second duration.

In addition, when the PPG signal needs to be continuously obtained, the processor 101 may further determine, based on the normalized distance, the second duration corresponding to the PPG signal that is continuously obtained. In other words, in this case, a value of the second duration is related to the similarity.

For example, the processor 101 may determine the second duration based on correspondences between intervals corresponding to a plurality of normalized distances and a plurality of durations. For example, the correspondences between intervals corresponding to normalized distances and durations may be shown in Table 1.

**Table 1**

| Interval corresponding to a normalized distance | 0 to 0.3 | 0.3 to 0.5 | 0.5 to 0.7 | 0.7 to 0.9 | 0.9 to 1 |
|---|---|---|---|---|---|
| Duration | 0 seconds | 15 seconds | 30 seconds | 45 seconds | 60 seconds |

For example, if the normalized distance between the PPG signals corresponding to the foregoing three pulse cycles and the 6^{th} set of PPG signals falls within the corresponding interval of 0 to 0.3, the processor 101 may determine that the PPG signal does not need to be obtained and collected. Alternatively, if the normalized distance between the PPG signals corresponding to the foregoing three pulse cycles and the 6^{th} set of PPG signals falls within the corresponding interval of 0.7 to 0.9, the processor 101 may determine that the PPG signal needs to be continuously obtained, and may determine that a PPG signal of 45 seconds needs to be continuously obtained. Further, the processor 101 continuously obtains the PPG signal of 45 seconds. In this case, the PPG signal of 45 seconds that is continuously obtained and the PPG signals corresponding to the foregoing three pulse cycles jointly form the 31^{st} set of PPG signals.

It should be noted that, the foregoing solution is merely used as an example for description, and does not constitute a limitation on this disclosure. A quantity of sets of PPG signals collected in a fixed duration based collection manner and a quantity of sets of PPG signals collected in a manner of flexibly determining a collection duration are not limited in this disclosure.

The following describes the method for determining, based on the k-nearest neighbor classification algorithm, the most possible category of the PPG signals corresponding to the foregoing three pulse cycles.

When the most possible category of the PPG signals corresponding to the foregoing three pulse cycles is determined, normalized distances between the pulse head wave peaks, the dicrotic wave heights, and the durations of the rising periods of the PPG signals corresponding to the three pulse cycles and pulse head wave peaks, dicrotic wave heights, and durations of rising periods of the first 30 sets of PPG signals may be separately determined. A total of 30 normalized distances are obtained. For ease of description, the 30 normalized distances are denoted as a normalized distance 1 to a normalized distance 30.

The foregoing 30 normalized distances are sorted in ascending order. Based on the order of the foregoing 30 normalized distances, several sets of PPG signals corresponding to the first several normalized distances are selected from the foregoing 30 sets of PPG signals. For example, 10 sets of PPG signals corresponding to the first 10 normalized distances are selected from the foregoing 30 sets of PPG signals.

In the 10 selected sets of PPG signals, it is assumed that three sets of PPG signals are of the category 1, five sets of PPG signals are of the category 2, and two sets of PPG signals are of the category 3. Because in the foregoing 10 sets of PPG signals, a quantity of sets of the category 2 is the largest, it may be determined that the most possible category of the PPG signals corresponding to the foregoing three pulse cycles is the category 2.

It should be noted that after the category of the 31^{st} set of PPG signals is determined, before the 32^{nd} set of PPG signals is collected, the 31^{st} set of PPG signals may be added to the category of the 31^{st} set of PPG signals. For example, it is determined that the 31^{st} set of PPG signals is of the category 2, so that the 31^{st} set of PPG signals may be added to the category 2. In other words, before the 31^{st} set of PPG signals is added to the category 2, the PPG signals of the category 2 include: the 2^{nd} set, the 6^{th} set, the 7^{th} set, the 11^{th} set, the 14^{th} set, the 17^{th} set, and the 18^{th} set of PPG signals. After the 31^{st} set of PPG signals is added to the category 2, PPG signals of the category 2 include: the 2^{nd} set, the 6^{th} set, the 7^{th} set, the 11^{th} set, the 14^{th} set, the 17^{th} set, the 18^{th} set, and the 31^{st} set of PPG signals.

After the 31^{st} set of PPG signals is added to the category 2, and before the 32^{nd} set of PPG signals is collected, a center of the plurality of sets of PPG signals of the category 2 may be re-determined. By analogy, when the 32^{nd} to 50^{th} sets of PPG signals are obtained, after a category of each set of PPG signals is determined and before a next set of PPG signals is obtained, the set of PPG signals may be added to the corresponding category, and a center of a plurality of sets of PPG signals of the category may be re-determined. The following describes a method for determining the center PPG signal of the plurality of sets of PPG signals of the category 2.

When the center PPG signal of the plurality of sets of PPG signals of the category 2 is determined, the 2^{nd} set, the 6^{th} set, the 7^{th} set, the 11^{th} set, the 14^{th} set, the 17^{th} set, and the 18^{th} set of PPG signals of the category 2 may be traversed. For example, for the 2^{nd} set of PPG signals, normalized distances between pulse head wave peaks, dicrotic wave heights, and durations of rising periods of the 6^{th} set, the 7^{th} set, the 11^{th} set, the 14^{th} set, the 17^{th} set, and the 18^{th} set of PPG signals and a pulse head wave peak, a dicrotic wave height, and a duration of a rising period of the 2^{nd} set of PPG signals are separately calculated. Six normalized distances are obtained, and a sum of the six normalized distances is determined. For the 6^{th} set of PPG signals, a sum is also obtained. By analogy, seven sums are finally obtained. A PPG signal corresponding to a maximum value in the seven sums is determined as the center of the plurality of sets of PPG signals of the category 2. For example, if the sum corresponding to the foregoing 6^{th} set of PPG signals is the maximum value in the seven sums, the 6^{th} set of PPG signals is the foregoing center of the plurality of sets of PPG signals of the category 2.

In this disclosure, one set of PPG signals may correspond to a plurality of pulses of the user, and one pulse cycle of the user corresponds to a PPG signal segment in one set of PPG signals. In other words, one set of PPG signals may include a plurality of PPG signal segments. A feature of each set of PPG signals may be a result of taking an average value or taking a median value of a plurality of features corresponding to the plurality of PPG signal segments. For example, if one set of PPG signals includes three PPG signal segments, each PPG signal segment includes three features: a pulse head wave peak, a dicrotic wave height, and a duration of a rising period. In this case, the features of the set of PPG signals may include a result obtained by taking an average value of three pulse head wave peaks, a result obtained by taking an average value of three dicrotic wave heights, and a result obtained by taking an average value of three dicrotic wave heights. Correspondingly, the features of the PPG signals corresponding to the foregoing three pulses cycles are a result of taking an average value or taking a median value of the three features corresponding to the three PPG signal segments. It should be noted that the duration of the rising period in this disclosure may be understood as: a time length required to rise from a minimum value to a maximum value of a waveform of each PPG signal segment.

It should be noted that, for a method for determining features of a set of PPG signals based on a plurality of features of a plurality of PPG signal segments, the foregoing listed method for taking an average value or taking a median value is merely used as an example for description, and does not constitute a limitation on this disclosure.

In this disclosure, whether the generated target model 2 is available may be determined in the following manners. In other words, whether the generated target model 2 meets measurement accuracy of the blood pressure value is determined. If the generated target model meets the measurement accuracy of the blood pressure value, it indicates that the target model 2 is successfully generated. In this case, the processor 101 may prompt the user to stop triggering the instruction for measuring blood pressure through the first measurement component 102.

### Manner 1

The processor 101 may determine, based on a value of N, whether the target model 2 is available.

For example, when the value of N is greater than or equal to 50, the processor 101 may determine that the target model 2 is available.

### Manner 2

The processor 101 may determine, based on quantities of sets of PPG signals of the category 1, the category 2, and the category 3, that the target model 2 is available.

For example, the processor 101 may determine, based on a comparison result of quantities of sets of the category 1, the category 2, and the category 3 and a preset sixth threshold, whether the target model 2 is available.

If a value of the preset sixth threshold is 10, when the quantities of sets of the category 1, the category 2, and the category 3 are all greater than or equal to 10, the processor 101 may determine that the target model 2 is available.

### Manner 3

After generating the target model 2 based on the N sets of blood pressure values and the N sets of PPG signals, the processor 101 may prompt the user to trigger the instruction for measuring blood pressure through the first measurement component 102. After obtaining the blood pressure value collected through the first measurement component 102, the processor may compare the blood pressure value collected through the first measurement component 102 with a blood pressure value obtained through the target model 2. If an absolute value of a difference between the blood pressure value collected through the first measurement component 102 and the blood pressure value obtained through the target model 2 is less than or equal to a preset seventh threshold, the processor 101 may determine that the target model 2 is available.

For example, if a value of the preset seventh threshold is 10, when the absolute value of the difference between the blood pressure value collected through the first measurement component 102 and the blood pressure value obtained through the target model 2 is less than or equal to 10, the processor 101 may determine that the target model 2 is available.

Optionally, after obtaining the target model 2 based on the N sets of blood pressure values and the N sets of PPG signals, the processor 101 is further configured to: in a process of measuring the blood pressure of the user based on the target model 2, determine whether the target model 2 needs to be updated, and when determining that the target model 2 needs to be updated, prompt the user to trigger the instruction for measuring blood pressure.

For example, after collecting the first PPG signal, the processor 101 may determine a category of the first PPG signal. For ease of description, the category of the first PPG signal is denoted as a second category. A similarity between the first PPG signal and a center of a plurality of sets of PPG signals of the second category in second historical PPG signals is further determined. Based on the similarity and a quantity of sets of PPG signals of the second category in the second historical PPG signals, it is determined that the target model 2 is to be updated.

After collecting the first PPG signal, the processor 101 determines, based on the first PPG signal and the foregoing 50 sets of PPG signals, that the first PPG signal is of the second category, where the second category is one of the category 1 to the category 3; further determines a normalized distance between the first PPG signal and a center of a plurality of sets of PPG signals of the second category in the foregoing 50 sets of PPG signals; and finally determines, based on the normalized distance between the first PPG signal and the center PPG signal and a quantity of sets of PPG signals of the second category in the 50 sets of PPG signals, whether the target model 2 needs to be updated. For a method for determining a most possible category of the first PPG signal and determining the normalized distance between the first PPG signal and the center PPG signal by the processor 101, refer to the foregoing related descriptions. For brevity, details are not described herein again. The 50 sets of PPG signals herein correspond to the second historical PPG signals. For example, when the normalized distance between the first PPG signal and the center PPG signal is less than or equal to the preset fifth threshold, and a quantity of the plurality of sets of PPG signals of the second category is less than or equal to the preset sixth threshold, the processor 101 may determine that the target model 2 needs to be updated.

For example, if the preset fifth threshold is 0.3, the preset sixth threshold is 10, the normalized distance between the first PPG signal and the center PPG signal is 0.2, and the quantity of the plurality of sets of PPG signals of the second category is 8, the processor 101 may determine that the target model 2 needs to be updated.

When determining that the target model 2 needs to be updated, the processor 101 may prompt, by using a voice, through vibration, or by using a pop-up window, the user to trigger the instruction for measuring blood pressure through the first measurement component 102. Alternatively, the processor 101 may prompt, by controlling a display screen to blink, the user to trigger the instruction for measuring blood pressure.

After receiving the prompt, the user may trigger the instruction for measuring blood pressure through the first measurement component 102. For example, the user may tap a button for measuring blood pressure on the watch, to send, to the processor 101, the instruction for measuring blood pressure through the first measurement component 102, or the user may speak "Start to measure blood pressure". After receiving the instruction, triggered by the user, for measuring blood pressure through the first measurement component 102, the processor 101 may collect a second blood pressure value of the user through the first measurement component 102, and collect a second PPG signal corresponding to the second blood pressure value through the second measurement component 103.

After obtaining a plurality of second blood pressure values and a plurality of sets of second PPG signals, the processor 101 may update the target model 2 based on the plurality of second blood pressure values and the plurality of sets of second PPG signals. For a manner in which the processor 101 obtains the second blood pressure value and the second PPG signal, refer to the foregoing related descriptions. For brevity, details are not described herein again.

After the processor 101 prompts the user to trigger the instruction for measuring blood pressure through the first measurement component 102, the user may set the watch, so that the processor 101 may periodically collect the second blood pressure value of the user through the first measurement component 102. Alternatively, the user may set the watch, so that the processor 101 can continuously collect the second blood pressure value of the user through the first measurement component 102.

For example, after updating the target model 2, if the processor 101 still receives the instruction for measuring blood pressure through the first measurement component 102, after receiving the instruction for measuring blood pressure through the first measurement component 102, the processor 101 may compare a blood pressure value of the user collected through the first measurement component 102 with a blood pressure value of the user obtained through the updated target model 2. If the blood pressure value of the user collected through the first measurement component 102 is close to the blood pressure value of the user obtained through the updated target model 2, the processor 101 may prompt the user to stop triggering the instruction for measuring blood pressure. For example, the processor 101 may prompt, by using a voice, through vibration, or by using a pop-up window, the user to stop triggering the instruction for measuring blood pressure.

It should be noted that, the processor 101 may alternatively determine, based on time in which the target model 2 is not updated, whether to update the target model 2. For example, when the processor 101 detects that the target model 2 has not been updated for a preset duration, the processor 101 may determine that the target model 2 needs to be updated.

For example, the preset duration may be one day. When the processor 101 detects that the target model 2 has not been updated for more than one day, the processor 101 may determine that the target model 2 needs to be updated.

In this disclosure, to improve accuracy of the PPG signal of the user collected through the second measurement component 103, the processor 101 may prompt, when the second measurement component 103 collects the PPG signal, the user to keep the wrist part still. For example, the processor 101 may prompt, by using a voice, through vibration, or by using a pop-up window, the user to keep the wrist part still.

Based on the blood pressure measurement apparatus provided in this disclosure, the target model 2 is obtained by model training based on the N sets of blood pressure values of the user (where the N sets of blood pressure values are obtained through the first measurement component 102, and accuracy of the blood pressure values obtained through the first measurement component 102 is high) and the N sets of PPG signals in one-to-one correspondence with the N sets of blood pressure values. Therefore, when the blood pressure value of the user is subsequently determined by the target model 2 based on the PPG signal of the user, an obtained measurement result of the blood pressure value is more accurate.

In addition, the second measurement component configured to collect the PPG signal is integrated with the first measurement component, so that the N sets of blood pressure values and the N sets of PPG signals in one-to-one correspondence with the N sets of blood pressure values that are used to generate the target model 2 can be obtained more conveniently.

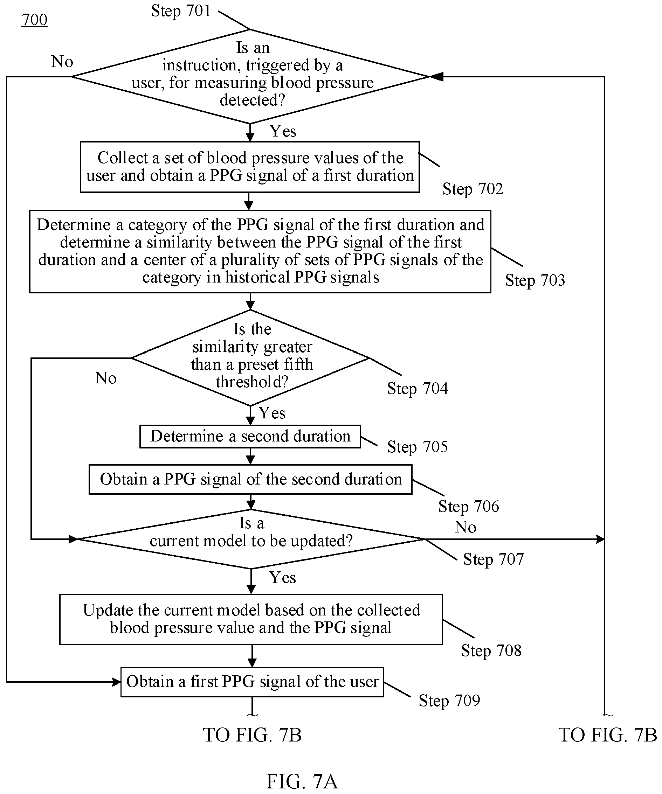

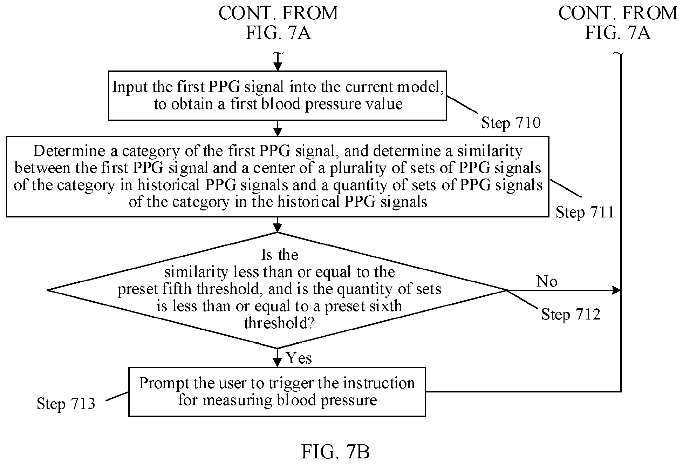

This disclosure further provides a blood pressure measurement method. The method is applied to the foregoing blood pressure measurement apparatus. FIG. 7A and FIG. 7B are an example flowchart of a method 700. The following describes the method 700 by using an example in which a target model 1 is pre-configured in a processor 101.

Step 701. The processor 101 detects that whether a user triggers an instruction for measuring blood pressure through a first measurement component 102.

If the processor 101 detects the instruction, triggered by the user, for measuring blood pressure through the first measurement component 102, the processor 101 performs step 702. If the processor 101 does not detect the instruction, triggered by the user, for measuring blood pressure through the first measurement component 102, the processor 101 performs step 709. It should be noted that if the processor 101 detects the instruction, triggered by the user, for measuring blood pressure through the first measurement component 102, the instruction for measuring blood pressure herein may be triggered by the user in the following two cases.

Case 1: In a process in which the target model 1 is updated, the user triggers the instruction for measuring blood pressure through the first measurement component 102.

Case 2: After a target model 2 is obtained, when the target model 2 needs to be updated, the user triggers the instruction for measuring blood pressure through the first measurement component 102.

Step 702. The processor 101 collects a set of blood pressure values of the user, and obtains a PPG signal of a first duration.

When detecting the instruction, triggered by the user, for measuring blood pressure through the first measurement component 102, the processor 101 collects the set of blood pressure values of the user through the first measurement component 102. When obtaining a set of PPG signals corresponding to the blood pressure value, the processor 101 may first obtain the PPG signal of the first duration.

Step 703. The processor 101 determines a category of the PPG signal of the first duration, and determines a similarity between the PPG signal of the first duration and a center of a plurality of sets of PPG signals of the category corresponding to the PPG signal of the first duration in historical PPG signals.

After obtaining the PPG signal of the first duration, the processor 101 may determine the category of the PPG signal of the first duration, and determine the similarity between the PPG signal of the first duration and the center of the plurality of sets of PPG signals of the category corresponding to the PPG signal of the first duration in the historical PPG signals. The historical PPG signals herein may be understood as: all PPG signals that have been obtained by the processor 101 before the processor 101 obtains the PPG signal of the first duration and that correspond to the blood pressure value of the user obtained through the first measurement component 102. For a specific method for determining the category of the PPG signal of the first duration and determining the similarity between the PPG signal of the first duration and the center of the plurality of sets of PPG signals of the category in the historical PPG signals by the processor 101, refer to the foregoing related descriptions. For brevity, details are not described herein again.

Step 704. The processor 101 determines whether the similarity between the PPG signal of the first duration and the center of the plurality of sets of PPG signals of the category corresponding to the PPG signal of the first duration in the historical PPG signals is greater than a preset fifth threshold. If the similarity between the PPG signal of the first duration and the center of the plurality of sets of PPG signals of the category corresponding to the PPG signal of the first duration in the historical PPG signals is greater than the preset fifth threshold, the processor 101 performs step 705. Alternatively, if the similarity between the PPG signal of the first duration and the center of the plurality of sets of PPG signals of the category corresponding to the PPG signal of the first duration in the historical PPG signals is less than or equal to the preset fifth threshold, the processor 101 performs step 707. In this case, the PPG signal of the first duration obtained in step 702 is a set of PPG signals corresponding to the blood pressure value in step 702.

Step 705. The processor 101 determines a second duration.

If the processor 101 determines that the similarity between the PPG signal of the first duration and the center of the PPG signals of the category in the historical PPG signals is greater than the preset fifth threshold, the processor 101 may determine that a PPG signal needs to be continuously obtained, and may determine that a PPG signal of the second duration needs to be continuously collected. For a specific method for determining whether the PPG signal needs to be continuously obtained and determining the second duration when the PPG signal needs to be continuously obtained, refer to the foregoing related descriptions. For brevity, details are not described herein again.

Step 706. The processor 101 obtains the PPG signal of the second duration.

After determining that the PPG signal of the second duration needs to be continuously collected, the processor 101 may continuously collect the PPG signal of the second duration. In this case, the PPG signal of the second duration that is continuously obtained and the PPG signal of the first duration that is obtained in step 702 form a set of PPG signals corresponding to the blood pressure value in step 702. For a specific method for obtaining the PPG signal of the first duration and the PPG signal of the second duration by the processor 101, refer to the foregoing related descriptions. For brevity, details are not described herein again.

Step 707. The processor 101 determines whether to update a current model.

After obtaining the blood pressure value of the user and the set of PPG signals corresponding to the blood pressure value, the processor 101 may determine to update the current model. For example, the processor 101 may determine, based on the accumulatively obtained blood pressure value and the PPG signal corresponding to the blood pressure value, whether to update the current model. For example, if the processor 101 has accumulatively obtained a specific quantity of blood pressure values and PPG signals, the processor 101 may perform step 708; otherwise, perform step 701.

For the current model, before step 707 is performed, if the processor 101 has not updated the pre-configured target model 1, the current model herein is the target model 1. Alternatively, before step 707 is performed, if the processor 101 has updated the target model 1, the current model herein is the target model 2. Alternatively, before step 707 is performed, if the processor 101 has updated the target model 2, the current model herein is an updated target model 2.

Step 708. The processor 101 updates the current model based on the collected blood pressure value and the PPG signal.

The processor 101 may update the current model based on the collected plurality of blood pressure values and the plurality of sets of PPG signals. For a specific method for updating the current model, refer to the foregoing related description. For brevity, details are not described herein again. Step 709. The processor 101 collects a first PPG signal through a second measurement component 103.

Step 710. The processor 101 inputs the first PPG signal into the current model, to obtain a first blood pressure value.

Step 711. The processor 101 determines a category of the first PPG signal, and determines a similarity between the first PPG signal and a center of a plurality of sets of PPG signals of the category in historical PPG signals and a quantity of sets of PPG signals of the category in the historical PPG signals.

After obtaining the first PPG signal, the processor 101 may determine the category of the first PPG signal, and determine the similarity between the first PPG signal and the center of the PPG signal of the category in the historical PPG signals. The historical PPG signals herein may be understood as: all PPG signals that have been obtained by the processor 101 before the processor 101 obtains the first PPG signal and that correspond to the blood pressure value of the user obtained through the first measurement component 102. For a specific method for determining the category of the first PPG signal and determining the similarity between the first PPG signal and the center of the PPG signals of the category in the historical PPG signals by the processor 101, refer to the foregoing related descriptions. For brevity, details are not described herein again.

Step 712. The processor 101 determines whether the similarity between the first PPG signal and the center of the plurality of sets of PPG signals of the category corresponding to the first PPG signal in the historical PPG signals is less than or equal to the preset fifth threshold, and whether the quantity of sets of PPG signals of the category in the historical PPG signals is less than or equal to a preset sixth threshold.

If the similarity between the first PPG signal and the center of the plurality of sets of PPG signals of the category corresponding to the first PPG signal in the historical PPG signals is less than or equal to the preset fifth threshold, and the quantity of sets of PPG signals of the category in the historical PPG signals is less than or equal to the preset sixth threshold, the processor 101 performs step 713. Otherwise, the processor 101 performs step 701. The historical PPG signals herein may be understood as: all PPG signals that have been obtained by the processor 101 before the processor 101 obtains the first PPG signal.

Step 713. The processor 101 prompts the user to trigger the instruction for measuring blood pressure.

If the similarity between the first PPG signal and the center of the plurality of sets of PPG signals of the category corresponding to the first PPG signal in the historical PPG signals is less than or equal to the preset fifth threshold, and the quantity of sets of PPG signals of the category in the historical PPG signals is less than or equal to the preset sixth threshold, it indicates that the current model needs to be updated. In this case, the processor 101 may prompt the user to trigger the instruction for measuring blood pressure. After prompting the user to trigger the instruction for measuring blood pressure, the processor 101 jumps to step 701. For a specific method for prompting, by the processor 101, the user to trigger the instruction for measuring blood pressure, refer to the foregoing related descriptions. For brevity, details are not described herein again.

According to the blood pressure measurement method provided in this disclosure, the target model 2 is obtained by updating the target model 1 based on N sets of blood pressure values of the user (where the N sets of blood pressure values are obtained through the first measurement component 102, and accuracy of the blood pressure values obtained through the first measurement component 102 is high) and N sets of PPG signals in one-to-one correspondence with the N sets of blood pressure values. Therefore, compared with the target model 1, when the blood pressure value of the user is subsequently determined by the target model 2 based on the PPG signal of the user, an obtained measurement result of the blood pressure value is more accurate.

In addition, the second measurement component configured to collect the PPG signal is integrated with the first measurement component, so that the N sets of blood pressure values and the N sets of PPG signals in one-to-one correspondence with the N sets of blood pressure values that are used to update the target model 1 can be obtained more conveniently.

It should be noted that, in this disclosure, an operation performed when the user triggers the instruction for measuring blood pressure through the first measurement component 102 corresponds to a first operation.

This disclosure further provides a computer storage medium, including computer instructions. When the computer instructions are run on a blood pressure measurement apparatus, the blood pressure measurement apparatus is enabled to perform the blood pressure measurement method provided in this disclosure.

This disclosure further provides a computer program product. When the computer program product runs on a computer, the computer is enabled to perform the blood pressure measurement method provided in this disclosure.

All or some of the foregoing examples may be implemented by software, hardware, firmware, or any combination thereof. When the examples are implemented by software, all or some of the foregoing examples may be implemented in a form of a computer program product. The computer program product includes one or more computer instructions. When the computer program instructions are loaded or executed on the computer, all or some of the processes or the functions according to examples of this disclosure are generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any available medium accessible by a computer, or a data storage device, such as a server or a data center, integrating one or more available media. The available medium may be a magnetic medium (for example, a floppy disk, a hard disk, or a magnetic tape), an optical medium (for example, a DVD), or a semiconductor medium. The semiconductor medium may be a solid state drive (solid state drive, SSD).

A person of ordinary skill in the art may be aware that, in combination with the examples described in examples disclosed in this specification, units and algorithm steps can be implemented by electronic hardware or a combination of computer software and electronic hardware. Whether the functions are performed by hardware or software depends on particular applications and design constraints of the technical solutions.

It may be clearly understood by a person skilled in the art that, for the purpose of convenient and brief description, for a detailed working process of the foregoing system, apparatus, and unit, refer to a corresponding process in the foregoing method examples. Details are not described herein again.

In several examples provided in examples of this disclosure, it should be understood that the disclosed system, apparatus, and method may be implemented in other manners. For example, the described apparatus example is merely an example. For example, division into the units is merely logical function division and may be another division during actual implementation. For example, a plurality of units or components may be combined or integrated into another system, or some features may be ignored or not performed. In addition, the displayed or discussed mutual couplings or direct couplings or communication connections may be implemented by some interfaces. The indirect couplings or communication connections between the apparatuses or units may be implemented in an electronic form, a mechanical form, or another form.

The units described as separate parts may or may not be physically separate, and parts displayed as units may or may not be physical units, in other words, and may be located at one location, or may be distributed on a plurality of network units. Some or all of the units may be selected based on actual requirements to achieve the objectives of solutions of examples.

In addition, functional units in examples of this disclosure may be integrated into one processing unit, or each of the units may exist alone physically, or two or more units are integrated into one unit.

When the functions are implemented in the form of a software functional unit and sold or used as an independent product, the functions may be stored in a computer-readable storage medium. Based on such an understanding, the technical solutions of examples of this disclosure essentially, or the part contributing to the conventional technology, or a part of the technical solutions may be implemented in a form of a software product. The computer software product is stored in a storage medium, and includes several instructions for instructing a computer device (which may be a personal computer, a server, a network device, or the like) to perform all or some of the steps of the methods described in examples of this disclosure. The foregoing storage medium includes: any medium that can store program code, such as a USB flash drive, a removable hard disk, a read-only memory (Read-Only Memory, ROM), a random-access memory (Random Access Memory, RAM), a magnetic disk, or an optical disc.

## Claims

1. A blood pressure measurement apparatus, wherein the blood pressure measurement apparatus comprises a processor (101), a first measurement component (102), and a second measurement component (103); the first measurement component (102) comprises an inflatable part (1022), an airbag (1023), and an air pressure sensor (1024); the airbag (1023) is separately connected to the inflatable part (1022) and the air pressure sensor (1024); the second measurement component (103) comprises a light source (1031) and a photoplethysmogram, PPG, sensor (1032);
the first measurement component (102) is configured to collect a blood pressure value of a user;
the second measurement component (103) is configured to collect a PPG signal of the user;
the processor (101) is configured to: control the first measurement component (102) to collect N sets of blood pressure values of the user; control the second measurement component (103) to collect N sets of PPG signals corresponding to the N sets of blood pressure values; and generate a target model based on the N sets of blood pressure values and the N sets of PPG signals, wherein an input of the target model is the PPG signal, an output of the target model is the blood pressure value, and N is an integer greater than or equal to 2;
the processor (101) is further configured to: after generating the target model, control the second measurement component (103) to collect a first PPG signal of the user; and determine a first blood pressure value based on the target model and the first PPG signal; and
the processor (101) is further configured to: when an i^{th} set of PPG signals in the N sets of PPG signals is collected,
control the second measurement component (103) to obtain a PPG signal of a first duration;
determine a category of the PPG signal of the first duration;
determine a similarity between the PPG signal of the first duration and a center of the category of the PPG signal of the first duration in first historical PPG signals, wherein the first historical PPG signals comprise a PPG signal that has been obtained before the i^{th} set of PPG signals is obtained;
determine a second duration based on the similarity;
control the second measurement component (103) to collect a PPG signal of the second duration; and
combine the PPG signal of the first duration and the PPG signal of the second duration into the i^{th} set of PPG signals.

2. The blood pressure measurement apparatus according to claim 1, wherein
the processor (101) is further configured to: when 1^{st} to M^{th} sets of PPG signals in the N sets of PPG signals are collected, control the second measurement component (103) to obtain a PPG signal of a third duration, wherein M is an integer less than i.

3. The blood pressure measurement apparatus according to any one of claims 1 to 2, wherein
the processor (101) is further configured to: when determining to update the target model, prompt the user to trigger an instruction for measuring blood pressure through the first measurement component (102) and the second measurement component (103);
in response to a first operation of the user, control the first measurement component (102) to collect a second blood pressure value, and control the second measurement component (103) to collect a second PPG signal corresponding to the second blood pressure value; and
update the target model based on the second blood pressure value and the second PPG signal.

4. The blood pressure measurement apparatus according to any one of claims 1 to 3, wherein
the processor (101) is further configured to determine a category of the first PPG signal; and
the processor (101) is further configured to: determine a similarity between the first PPG signal and a center of PPG signals of the category of the first PPG signal in second historical PPG signals, and determine, based on the similarity and a quantity of sets of PPG signals of the category of the first PPG signal in the second historical PPG signals, whether the target model needs to be updated, wherein the second historical PPG signals comprise the N sets of PPG signals.

5. The blood pressure measurement apparatus according to any one of claims 1 to 3, wherein
the processor (101) is further configured to determine, based on a duration in which the target model is not updated, whether the target model needs to be updated.

6. The blood pressure measurement apparatus according to any one of claims 1 to 5, wherein
the inflatable part (1022) is configured to inflate the airbag (1023); and
the air pressure sensor (1024) is configured to collect a plurality of air pressure values of the airbag (1023), wherein a first air pressure value and a second air pressure value in the plurality of air pressure values are blood pressure values of the user, the first air pressure value is an air pressure value corresponding to a moment at which an oscillatory wave of air pressure in the airbag (1023) reaches a maximum value, the second air pressure value is an air pressure value corresponding to a moment at which the oscillatory wave reaches the maximum value multiplied by a, and a is greater than 0 and less than 1.

7. The blood pressure measurement apparatus according to any one of claims 1 to 6, wherein a difference between a moment at which the airbag (1023) starts to be inflated and a collection moment of the i^{th} set of PPG signals in the N sets of PPG signals is greater than or equal to a preset first threshold and is less than or equal to a preset second threshold, and the moment at which the airbag (1023) starts to be inflated is after the collection moment of the i^{th} set of PPG signals, or a difference between a collection moment of the i^{th} set of PPG signals in the N sets of PPG signals and a moment at which inflation of the airbag (1023) is stopped is greater than or equal to a preset third threshold and is less than or equal to a preset fourth threshold, and the moment at which inflation of the airbag (1023) is stopped is before the collection moment of the i^{th} set of PPG signals.

8. A blood pressure measurement method, wherein the method is applied to a blood pressure measurement apparatus; the blood pressure measurement apparatus comprises a first measurement component (102) and a second measurement component (103); the first measurement component (102) comprises an inflatable part (1022), an airbag (1023), and an air pressure sensor (1024); the airbag (1023) is separately connected to the inflatable part (1022) and the air pressure sensor (1024); the second measurement component (103) comprises a light source (1031) and a photoplethysmogram, PPG, sensor (1032); and the method comprises:
controlling the first measurement component (102) to collect N sets of blood pressure values of a user;
controlling the second measurement component (103) to collect N sets of PPG signals corresponding to the N sets of blood pressure values;
generating a target model based on the N sets of blood pressure values and the N sets of PPG signals, wherein an input of the target model is the PPG signal, an output of the target model is the blood pressure value, and N is an integer greater than or equal to 2;
after generating the target model, controlling the second measurement component to collect a first PPG signal of the user; and
determining a first blood pressure value based on the target model and the first PPG signal, wherein when an i^{th} set of PPG signals in the N sets of PPG signals is collected, the controlling the second measurement component (103) to collect N sets of PPG signals corresponding to the N sets of blood pressure values comprises:
controlling the second measurement component (103) to obtain a PPG signal of a first duration;
determining a category of the PPG signal of the first duration;
determining a similarity between the PPG signal of the first duration and a center of the category of the PPG signal of the first duration in first historical PPG signals, wherein the first historical PPG signals comprise a PPG signal that has been obtained before the i^{th} set of PPG signals is obtained;
determining a second duration based on the similarity;
controlling the second measurement component (103) to collect a PPG signal of the second duration; and
combining the PPG signal of the first duration and the PPG signal of the second duration into the i^{th} set of PPG signals.

9. The blood pressure measurement method according to claim 8, wherein when 1^{st} to M^{th} sets of PPG signals in the N sets of PPG signals are collected, the controlling the second measurement component (103) to collect N sets of PPG signals corresponding to the N sets of blood pressure values comprises:
controlling the second measurement component (103) to obtain a PPG signal of a third duration, wherein M is an integer less than i.

10. The blood pressure measurement method according to any one of claims 8 to 9, wherein the method further comprises:
when determining to update the target model, prompting the user to trigger an instruction for measuring blood pressure through the first measurement component (102) and the second measurement component (103);
in response to a first operation of the user, controlling the first measurement component (102) to collect a second blood pressure value, and controlling the second measurement component (103) to collect a second PPG signal corresponding to the second blood pressure value; and
updating the target model based on the second blood pressure value and the second PPG signal.

11. The blood pressure measurement method according to any one of claims 8 to 10, wherein the method further comprises:
determining a category of the first PPG signal; and
determining a similarity between the first PPG signal and a center of PPG signals of the category of the first PPG signal in second historical PPG signals, and determining, based on the similarity and a quantity of sets of PPG signals of the category of the first PPG signal in the second historical PPG signals, whether the target model needs to be updated, wherein
the second historical PPG signals comprise the N sets of PPG signals.

12. The blood pressure measurement method according to any one of claims 8 to 11, wherein the controlling the first measurement component (102) to collect N sets of blood pressure values of a user comprises:
controlling the inflatable part (1022) to inflate the airbag (1023); and
controlling the air pressure sensor (1024) to collect a plurality of air pressure values of the airbag (1023), wherein a first air pressure value and a second air pressure value in the plurality of air pressure values are a set of blood pressure values of the user, the first air pressure value is an air pressure value corresponding to a moment at which an oscillatory wave of air pressure in the airbag (1023) reaches a maximum value, the second air pressure value is an air pressure value corresponding to a moment at which the oscillatory wave reaches the maximum value multiplied by a, and a is greater than 0 and less than 1.

13. The blood pressure measurement method according to any one of claims 8 to 12, wherein a difference between a moment at which the airbag (1023) starts to be inflated and a collection moment of the i^{th} set of PPG signals in the N sets of PPG signals is greater than or equal to a preset first threshold and is less than or equal to a preset second threshold, and the moment at which the airbag (1023) starts to be inflated is after the collection moment of the i^{th} set of PPG signals, or a difference between a collection moment of the i^{th} set of PPG signals in the N sets of PPG signals and a moment at which inflation of the airbag (1023) is stopped is greater than or equal to a preset third threshold and is less than or equal to a preset fourth threshold, and the moment at which inflation of the airbag (1023) is stopped is before the collection moment of the i^{th} set of PPG signals.

14. A computer storage medium, comprising computer instructions, wherein when the computer instructions are run on a blood pressure measurement apparatus, the blood pressure measurement apparatus is enabled to perform the blood pressure measurement method according to any one of claims 8 to 13.

15. A computer program product, wherein when the computer program product runs on a computer, the computer is enabled to perform the blood pressure measurement method according to any one of claims 8 to 13.

## Patentansprüche

1. Vorrichtung zur Blutdruckmessung, wobei die Vorrichtung zur Blutdruckmessung einen Prozessor (101), eine erste Messkomponente (102) und eine zweite Messkomponente (103) umfasst; die erste Messkomponente (102) einen aufblasbaren Teil (1022), einen Airbag (1023) und einen Luftdrucksensor (1024) umfasst; der Airbag (1023) separat mit dem aufblasbaren Teil (1022) und dem Luftdrucksensor (1024) verbunden ist; die zweite Messkomponente (103) eine Lichtquelle (1031) und einen Photoplethysmogramm-Sensor, PPG-Sensor, (1032) umfasst;
die erste Messkomponente (102) dazu konfiguriert ist, einen Blutdruckwert eines Benutzers zu erfassen;
die zweite Messkomponente (103) dazu konfiguriert ist, ein PPG-Signal des Benutzers zu erfassen;
der Prozessor (101) dazu konfiguriert ist: die erste Messkomponente (102) zu steuern, um N Sätze von Blutdruckwerten des Benutzers zu erfassen; die zweite Messkomponente (103) zu steuern, um N Sätze von PPG-Signalen entsprechend den N Sätzen von Blutdruckwerten zu erfassen; und ein Zielmodell basierend auf den N Sätzen von Blutdruckwerten und den N Sätzen von PPG-Signalen zu generieren, wobei ein Eingang des Zielmodells das PPG-Signal ist, ein Ausgang des Zielmodells der Blutdruckwert ist und N eine Ganzzahl größer oder gleich 2 ist;
der Prozessor (101) ferner dazu konfiguriert ist: nach dem Generieren des Zielmodells die zweite Messkomponente (103) zu steuern, um ein erstes PPG-Signal des Benutzers zu erfassen; und einen ersten Blutdruckwert basierend auf dem Zielmodell und dem ersten PPG-Signal zu bestimmen; und
der Prozessor (101) ferner dazu konfiguriert ist: wenn ein i-ter Satz von PPG-Signalen in den N Sätzen von PPG-Signalen erfasst wird,
die zweite Messkomponente (103) zu steuern, um ein PPG-Signal einer ersten Dauer zu erlangen;
eine Kategorie des PPG-Signals der ersten Dauer zu bestimmen;
eine Ähnlichkeit zwischen dem PPG-Signal der ersten Dauer und einer Mitte der Kategorie des PPG-Signals der ersten Dauer in ersten historischen PPG-Signalen zu bestimmen, wobei die ersten historischen PPG-Signale ein PPG-Signal umfassen, das erlangt wurde, bevor der i-te Satz von PPG-Signalen erlangt wurde;
eine zweite Dauer basierend auf der Ähnlichkeit zu bestimmen;
die zweite Messkomponente (103) zu steuern, um ein PPG-Signal der zweiten Dauer zu erfassen; und
das PPG-Signal der ersten Dauer und das PPG-Signal der zweiten Dauer in den i-ten Satz von PPG-Signalen zu kombinieren.

2. Vorrichtung zur Blutdruckmessung nach Anspruch 1, wobei der Prozessor (101) ferner dazu konfiguriert ist: wenn der 1. bis M-te Satz von PPG-Signalen in den N Sätzen von PPG-Signalen erfasst sind, die zweite Messkomponente (103) zu steuern, um ein PPG-Signal einer dritten Dauer zu erlangen, wobei M eine Ganzzahl kleiner als i ist.

3. Vorrichtung zur Blutdruckmessung nach einem der Ansprüche 1 bis 2, wobei
der Prozessor (101) ferner dazu konfiguriert ist: beim Bestimmen des Aktualisierens des Zielmodells, den Benutzer aufzufordern, eine Anweisung zum Messen des Blutdrucks durch die erste Messkomponente (102) und die zweite Messkomponente (103) auszulösen;
als Reaktion auf eine erste Operation des Benutzers die erste Messkomponente (102) zu steuern, um einen zweiten Blutdruckwert zu erfassen, und die zweite Messkomponente (103) zu steuern, um ein zweites PPG-Signal entsprechend dem zweiten Blutdruckwert zu erfassen; und
das Zielmodell basierend auf dem zweiten Blutdruckwert und dem zweiten PPG-Signal zu aktualisieren.

4. Vorrichtung zur Blutdruckmessung nach einem der Ansprüche 1 bis 3, wobei
der Prozessor (101) ferner dazu konfiguriert ist, eine Kategorie des ersten PPG-Signals zu bestimmen; und
der Prozessor (101) ferner dazu konfiguriert ist: eine Ähnlichkeit zwischen dem ersten PPG-Signal und einer Mitte von PPG-Signalen der Kategorie des ersten PPG-Signals in zweiten historischen PPG-Signalen zu bestimmen und basierend auf der Ähnlichkeit und einer Menge von Sätzen von PPG-Signalen der Kategorie des ersten PPG-Signals in den zweiten historischen PPG-Signalen zu bestimmen, ob das Zielmodell aktualisiert werden muss, wobei die zweiten historischen PPG-Signale die N Sätze von PPG-Signalen umfassen.

5. Vorrichtung zur Blutdruckmessung nach einem der Ansprüche 1 bis 3, wobei
der Prozessor (101) ferner dazu konfiguriert ist, basierend auf einer Dauer, in welcher das Zielmodell nicht aktualisiert wird, zu bestimmen, ob das Zielmodell aktualisiert werden muss.

6. Vorrichtung zur Blutdruckmessung nach einem der Ansprüche 1 bis 5, wobei
der aufblasbare Teil (1022) dazu konfiguriert ist, den Airbag (1023) aufzublasen; und
der Luftdrucksensor (1024) dazu konfiguriert ist, eine Vielzahl von Luftdruckwerten des Airbags (1023) zu erfassen, wobei ein erster Luftdruckwert und ein zweiter Luftdruckwert in der Vielzahl von Luftdruckwerten Blutdruckwerte des Benutzers sind, der erste Luftdruckwert ein Luftdruckwert entsprechend einem Zeitpunkt ist, zu welchem eine oszillierende Welle des Luftdrucks in dem Airbag (1023) einen Maximalwert erreicht, der zweite Luftdruckwert ein Luftdruckwert entsprechend einem Zeitpunkt ist, zu welchem die oszillierende Welle den Maximalwert multipliziert mit a erreicht, und a größer als 0 und kleiner als 1 ist.

7. Vorrichtung zur Blutdruckmessung nach einem der Ansprüche 1 bis 6, wobei eine Differenz zwischen einem Zeitpunkt, zu welchem mit dem Aufblasen des Airbags (1023) begonnen wird, und einem Erfassungszeitpunkt des i-ten Satzes von PPG-Signalen in den N Sätzen von PPG-Signalen größer oder gleich einem voreingestellten ersten Schwellenwert ist und kleiner oder gleich einem voreingestellten zweiten Schwellenwert ist, und der Zeitpunkt, zu welchem mit dem Aufblasen des Airbags (1023) begonnen wird, nach dem Erfassungszeitpunkt des i-ten Satzes von PPG-Signalen liegt, oder eine Differenz zwischen einem Erfassungszeitpunkt des i-ten Satzes von PPG-Signalen in den N Sätzen von PPG-Signalen und einem Zeitpunkt, zu welchem das Aufblasen des Airbags (1023) gestoppt wird, größer oder gleich einem voreingestellten dritten Schwellenwert und kleiner oder gleich einem voreingestellten vierten Schwellenwert ist, und der Zeitpunkt, zu welchem das Aufblasen des Airbags (1023) gestoppt wird, vor dem Erfassungszeitpunkt des i-ten Satzes von PPG-Signalen liegt.

8. Verfahren zur Blutdruckmessung, wobei das Verfahren auf eine Vorrichtung zur Blutdruckmessung angewendet wird; die Vorrichtung zur Blutdruckmessung eine erste Messkomponente (102) und eine zweite Messkomponente (103) umfasst; die erste Messkomponente (102) einen aufblasbaren Teil (1022), einen Airbag (1023) und einen Luftdrucksensor (1024) umfasst; der Airbag (1023) separat mit dem aufblasbaren Teil (1022) und dem Luftdrucksensor (1024) verbunden ist; die zweite Messkomponente (103) eine Lichtquelle (1031) und einen Photoplethysmogramm-Sensor, PPG-Sensor, (1032) umfasst; und das Verfahren Folgendes umfasst:
Steuern der ersten Messkomponente (102), um N Sätze von Blutdruckwerten eines Benutzers zu erfassen;
Steuern der zweiten Messkomponente (103), um N Sätze von PPG-Signalen entsprechend den N Sätzen von Blutdruckwerten zu erfassen;
Generieren eines Zielmodells basierend auf den N Sätzen von Blutdruckwerten und den N Sätzen von PPG-Signalen, wobei ein Eingang des Zielmodells das PPG-Signal ist, ein Ausgang des Zielmodells der Blutdruckwert ist und N eine Ganzzahl größer oder gleich 2 ist;
nach dem Generieren des Zielmodells, Steuern der zweiten Messkomponente, um ein erstes PPG-Signal des Benutzers zu erfassen; und
Bestimmen eines ersten Blutdruckwerts basierend auf dem Zielmodell und dem ersten PPG-Signal, wobei, wenn ein i-ter Satz von PPG-Signalen in den N Sätzen von PPG-Signalen erfasst wird,
das Steuern der zweiten Messkomponente (103) zum Erfassen von N Sätzen von PPG-Signalen entsprechend den N Sätzen von Blutdruckwerten Folgendes umfasst:
Steuern der zweiten Messkomponente (103), um ein PPG-Signal einer ersten Dauer zu erlangen;
Bestimmen einer Kategorie des PPG-Signals der ersten Dauer;
Bestimmen einer Ähnlichkeit zwischen dem PPG-Signal der ersten Dauer und einer Mitte der Kategorie des PPG-Signals der ersten Dauer in ersten historischen PPG-Signalen, wobei die ersten historischen PPG-Signale ein PPG-Signal umfassen, das erlangt wurde, bevor der i-te Satz von PPG-Signalen erlangt wurde;
Bestimmen einer zweiten Dauer basierend auf der Ähnlichkeit;
Steuern der zweiten Messkomponente (103), um ein PPG-Signal der zweiten Dauer zu erfassen; und
Kombinieren des PPG-Signals der ersten Dauer und des PPG-Signals der zweiten Dauer in den i-ten Satz von PPG-Signalen.

9. Verfahren zur Blutdruckmessung nach Anspruch 8, wobei, wenn der 1. bis M-te Satz von PPG-Signalen in den N Sätzen von PPG-Signalen erfasst werden, das Steuern der zweiten Messkomponente (103) zum Erfassen von N Sätzen von PPG-Signalen entsprechend den N Sätzen von Blutdruckwerten Folgendes umfasst:
Steuern der zweiten Messkomponente (103), um ein PPG-Signal einer dritten Dauer zu erlangen, wobei M eine Ganzzahl kleiner als i ist.

10. Verfahren zur Blutdruckmessung nach einem der Ansprüche 8 bis 9, wobei das Verfahren ferner Folgendes umfasst:
beim Bestimmen des Aktualisierens des Zielmodells, Auffordern des Benutzers, eine Anweisung zum Messen des Blutdrucks durch die erste Messkomponente (102) und die zweite Messkomponente (103) auszulösen;
als Reaktion auf eine erste Operation des Benutzers, Steuern der ersten Messkomponente (102), um einen zweiten Blutdruckwert zu erfassen, und Steuern der zweiten Messkomponente (103), um ein zweites PPG-Signal entsprechend dem zweiten Blutdruckwert zu erfassen; und
Aktualisieren des Zielmodells basierend auf dem zweiten Blutdruckwert und dem zweiten PPG-Signal.

11. Verfahren zur Blutdruckmessung nach einem der Ansprüche 8 bis 10, wobei das Verfahren ferner Folgendes umfasst:
Bestimmen einer Kategorie des ersten PPG-Signals; und
Bestimmen einer Ähnlichkeit zwischen dem ersten PPG-Signal und einer Mitte von PPG-Signalen der Kategorie des ersten PPG-Signals in zweiten historischen PPG-Signalen, und Bestimmen, basierend auf der Ähnlichkeit und einer Menge von Sätzen von PPG-Signalen der Kategorie des ersten PPG-Signals in den zweiten historischen PPG-Signalen, ob das Zielmodell aktualisiert werden muss, wobei
die zweiten historischen PPG-Signale die N Sätze von PPG-Signalen umfassen.

12. Verfahren zur Blutdruckmessung nach einem der Ansprüche 8 bis 11, wobei das Steuern der ersten Messkomponente (102) zum Erfassen von N Sätzen von Blutdruckwerten eines Benutzers Folgendes umfasst:
Steuern des aufblasbaren Teils (1022), um den Airbag (1023) aufzublasen; und
Steuern des Luftdrucksensors (1024) zum Erfassen einer Vielzahl von Luftdruckwerten des Airbags (1023), wobei ein erster Luftdruckwert und ein zweiter Luftdruckwert in der Vielzahl von Luftdruckwerten ein Satz von Blutdruckwerten des Benutzers sind,
der erste Luftdruckwert ein Luftdruckwert entsprechend einem Zeitpunkt ist, zu welchem eine oszillierende Welle des Luftdrucks in dem Airbag (1023) einen Maximalwert erreicht, der zweite Luftdruckwert ein Luftdruckwert entsprechend einem Zeitpunkt ist, zu welchem die oszillierende Welle den Maximalwert multipliziert mit a erreicht, und a größer als 0 und kleiner als 1 ist.

13. Verfahren zur Blutdruckmessung nach einem der Ansprüche 8 bis 12, wobei eine Differenz zwischen einem Zeitpunkt, zu welchem mit dem Aufblasen des Airbags (1023) begonnen wird, und einem Erfassungszeitpunkt des i-ten Satzes von PPG-Signalen in den N Sätzen von PPG-Signalen größer oder gleich einem voreingestellten ersten Schwellenwert und kleiner oder gleich einem voreingestellten zweiten Schwellenwert ist, und der Zeitpunkt, zu welchem mit dem Aufblasen des Airbags (1023) begonnen wird, nach dem Erfassungszeitpunkt des i-ten Satzes von PPG-Signalen liegt, oder eine Differenz zwischen einem Erfassungszeitpunkt des i-ten Satzes von PPG-Signalen in den N Sätzen von PPG-Signalen und einem Zeitpunkt, zu welchem das Aufblasen des Airbags (1023) gestoppt wird, größer oder gleich einem voreingestellten dritten Schwellenwert und kleiner oder gleich einem voreingestellten vierten Schwellenwert ist, und der Zeitpunkt, zu welchem das Aufblasen des Airbags (1023) gestoppt wird, vor dem Erfassungszeitpunkt des i-ten Satzes von PPG-Signalen liegt.

14. Computerspeichermedium, umfassend Computeranweisungen, wobei, wenn die Computeranweisungen auf einer Vorrichtung zur Blutdruckmessung ausgeführt werden, die Vorrichtung zur Blutdruckmessung in der Lage ist, das Verfahren zur Blutdruckmessung nach einem der Ansprüche 8 bis 13 durchzuführen.

15. Computerprogrammprodukt, wobei, wenn das Computerprogrammprodukt auf einem Computer ausgeführt wird, der Computer in der Lage ist, das Verfahren zur Blutdruckmessung nach einem der Ansprüche 8 bis 13 durchzuführen.

## Revendications

1. Appareil de mesure de la pression artérielle, dans lequel l'appareil de mesure de la pression artérielle comprend un processeur (101), un premier composant de mesure (102), et un second composant de mesure (103) ; le premier composant de mesure (102) comprend une partie gonflable (1022), un coussin gonflable (1023), et un capteur de pression d'air (1024) ; le coussin gonflable (1023) est relié séparément à la partie gonflable (1022) et au capteur de pression d'air (1024) ; le second composant de mesure (103) comprend une source lumineuse (1031) et un capteur de photopléthysmogramme (PPG) (1032) ;
le premier composant de mesure (102) est configuré pour collecter une valeur de pression artérielle d'un utilisateur ;
le second composant de mesure (103) est configuré pour collecter un signal PPG de l'utilisateur ;
le processeur (101) est configuré pour : commander le premier composant de mesure (102) pour collecter N ensembles de valeurs de pression artérielle de l'utilisateur ; commander le second composant de mesure (103) pour collecter N ensembles de signaux PPG correspondant aux N ensembles de valeurs de pression artérielle ; et générer un modèle cible sur la base des N ensembles de valeurs de pression artérielle et les N ensembles de signaux PPG, dans lequel une entrée du modèle cible est le signal PPG, une sortie du modèle cible est la valeur de pression artérielle, et N est un entier supérieur ou égal à 2 ;
le processeur (101) est également configuré pour : après avoir généré le modèle cible, commander le second composant de mesure (103) pour collecter un premier signal PPG de l'utilisateur ; et déterminer une première valeur de pression artérielle sur la base du modèle cible et du premier signal PPG ; et
le processeur (101) est également configuré pour : lorsqu'un i^{ième} ensemble de signaux PPG dans les N ensembles de signaux PPG est collecté,
commander le second composant de mesure (103) pour obtenir un signal PPG d'une première durée ;
déterminer une catégorie du signal PPG de la première durée ;
déterminer une similarité entre le signal PPG de la première durée et un centre de la catégorie du signal PPG de la première durée dans les premiers signaux PPG historiques, dans lequel les premiers signaux PPG historiques comprennent un signal PPG qui a été obtenu avant que le i^{ième} ensemble de signaux PPG ne soit obtenu ;
déterminer une deuxième durée en fonction de la similarité ; commander le second composant de mesure (103) pour collecter un signal PPG de la deuxième durée ; et
combiner le signal PPG de la première durée et le signal PPG de la deuxième durée dans le i^{ième} ensemble de signaux PPG.

2. Appareil de mesure de la pression artérielle selon la revendication 1, dans lequel
le processeur (101) est également configuré pour : lorsque les 1^{er} à M^{ième} ensembles de signaux PPG dans les N ensembles de signaux PPG sont collectés, commander le second composant de mesure (103) pour obtenir un signal PPG d'une troisième durée, dans lequel M est un entier inférieur à i.

3. Appareil de mesure de la pression artérielle selon l'une quelconque des revendications 1 à 2, dans lequel
le processeur (101) est également configuré pour : lors de la détermination de la mise à jour du modèle cible, inviter l'utilisateur à déclencher une instruction de mesure de la pression artérielle via le premier composant de mesure (102) et le second composant de mesure (103) ;
en réponse à une première opération de l'utilisateur, commander le premier composant de mesure (102) pour collecter une seconde valeur de pression artérielle, et commander le second composant de mesure (103) pour collecter un second signal PPG correspondant à la seconde valeur de pression artérielle ; et
mettre à jour le modèle cible sur la base de la seconde valeur de pression artérielle et du second signal PPG.

4. Appareil de mesure de la pression artérielle selon l'une quelconque des revendications 1 à 3, dans lequel
le processeur (101) est également configuré pour déterminer une catégorie du premier signal PPG ; et
le processeur (101) est également configuré pour : déterminer une similarité entre le premier signal PPG et un centre de signaux PPG de la catégorie du premier signal PPG dans des seconds signaux PPG historiques, et déterminer, sur la base de la similarité et d'une quantité d'ensembles de signaux PPG de la catégorie du premier signal PPG dans les seconds signaux PPG historiques, si le modèle cible doit être mis à jour, dans lequel les seconds signaux PPG historiques comprennent les N ensembles de signaux PPG.

5. Appareil de mesure de la pression artérielle selon l'une quelconque des revendications 1 à 3, dans lequel
le processeur (101) est également configuré pour déterminer, sur la base d'une durée pendant laquelle le modèle cible n'est pas mis à jour, si le modèle cible doit être mis à jour.

6. Appareil de mesure de la pression artérielle selon l'une quelconque des revendications 1 à 5, dans lequel
la partie gonflable (1022) est configurée pour gonfler le coussin gonflable (1023) ; et
le capteur de pression d'air (1024) est configuré pour collecter une pluralité de valeurs de pression d'air du coussin gonflable (1023), dans lequel une première valeur de pression d'air et une seconde valeur de pression d'air dans la pluralité de valeurs de pression d'air sont des valeurs de pression artérielle de l'utilisateur, la première valeur de pression d'air est une valeur de pression d'air correspondant à un moment pendant lequel une onde oscillatoire de pression d'air dans le coussin gonflable (1023) atteint une valeur maximale, la seconde valeur de pression d'air est une valeur de pression d'air correspondant à un moment pendant lequel l'onde oscillatoire atteint la valeur maximale multipliée par a, et a est supérieur à 0 et inférieur à 1.

7. Appareil de mesure de la pression artérielle selon l'une quelconque des revendications 1 à 6, dans lequel une différence entre un moment pendant lequel le coussin gonflable (1023) commence à se gonfler et un moment de collecte du i^{ième} ensemble de signaux PPG parmi les N ensembles de signaux PPG est supérieure ou égale à un premier seuil prédéfini et est inférieure ou égale à un deuxième seuil prédéfini, et le moment pendant lequel le coussin gonflable (1023) commence à se gonfler est postérieur au moment de collecte du i^{ième} ensemble de signaux PPG, ou une différence entre un moment de collecte du i^{ième} ensemble de signaux PPG parmi les N ensembles de signaux PPG et un moment pendant lequel le gonflage du coussin gonflable (1023) est arrêté est supérieure ou égale à un troisième seuil prédéfini et est inférieure ou égale à un quatrième seuil prédéfini, et le moment pendant lequel le gonflage du coussin gonflable (1023) est arrêté est antérieur au moment de la collecte du i^{ième} ensemble de signaux PPG.

8. Procédé de mesure de la pression artérielle, dans lequel le procédé s'applique à un appareil de mesure de la pression artérielle ; l'appareil de mesure de la pression artérielle comprend un premier composant de mesure (102) et un second composant de mesure (103) ; le premier composant de mesure (102) comprend une partie gonflable (1022), un coussin gonflable (1023) et un capteur de pression d'air (1024) ; le coussin gonflable (1023) est relié séparément à la partie gonflable (1022) et au capteur de pression d'air (1024) ; le second composant de mesure (103) comprend une source lumineuse (1031) et un capteur de photopléthysmogramme, PPG (1032) ; et le procédé comprend :
la commande du premier composant de mesure (102) pour collecter N ensembles de valeurs de pression artérielle d'un utilisateur ;
la commande du second composant de mesure (103) pour collecter N ensembles de signaux PPG correspondant aux N ensembles de valeurs de pression artérielle ;
la génération d'un modèle cible sur la base des N ensembles de valeurs de pression artérielle et des N ensembles de signaux PPG, dans lequel une entrée du modèle cible est le signal PPG, une sortie du modèle cible est la valeur de pression artérielle, et N est un entier supérieur ou égal à 2 ;
après avoir généré le modèle cible, la commande du second composant de mesure pour collecter un premier signal PPG de l'utilisateur ; et
la détermination d'une première valeur de pression artérielle sur la base du modèle cible et du premier signal PPG, dans lequel lorsqu'un i^{ième} ensemble de signaux PPG parmi les N ensembles de signaux PPG est collecté, la commande du second composant de mesure (103) pour collecter N ensembles de signaux PPG correspondant aux N ensembles de valeurs de pression artérielle comprend :
la commande du second composant de mesure (103) pour obtenir un signal PPG d'une première durée ;
la détermination d'une catégorie du signal PPG de la première durée ;
la détermination d'une similarité entre le signal PPG de la première durée et un centre de la catégorie du signal PPG de la première durée dans les premiers signaux PPG historiques, dans lequel les premiers signaux PPG historiques comprennent un signal PPG qui a été obtenu avant que le i^{ième} ensemble de signaux PPG ne soit obtenu ;
la détermination d'une deuxième durée sur la base de la similarité ;
la commande du second composant de mesure (103) pour collecter un signal PPG de la deuxième durée ; et
la combinaison du signal PPG de la première durée et le signal PPG de la deuxième durée dans le i^{ième} ensemble de signaux PPG.

9. Procédé de mesure de la pression artérielle selon la revendication 8, dans lequel, lorsque les 1^{er} à M^{ième} ensembles de signaux PPG parmi les N ensembles de signaux PPG sont collectés, la commande du second composant de mesure (103) pour collecter les N ensembles de signaux PPG correspondant aux N ensembles de valeurs de pression artérielle comprend :
la commande du second composant de mesure (103) pour obtenir un signal PPG d'une troisième durée, dans lequel M est un entier inférieur à i.

10. Procédé de mesure de la pression artérielle selon l'une quelconque des revendications 8 et 9, dans lequel le procédé comprend également :
lors de la détermination de la mise à jour du modèle cible, l'invitation de l'utilisateur à déclencher une instruction de mesure de la pression artérielle via le premier composant de mesure (102) et le second composant de mesure (103) ;
en réponse à une première opération de l'utilisateur, la commande du premier composant de mesure (102) pour collecter une seconde valeur de pression artérielle, et la commande du second composant de mesure (103) pour collecter un second signal PPG correspondant à la seconde valeur de pression artérielle ; et
la mise à jour du modèle cible sur la base de la seconde valeur de pression artérielle et du second signal PPG.

11. Procédé de mesure de la pression artérielle selon l'une quelconque des revendications 8 à 10, dans lequel le procédé comprend également :
la détermination d'une catégorie du premier signal PPG ; et
la détermination d'une similarité entre le premier signal PPG et un centre de signaux PPG de la catégorie du premier signal PPG dans les seconds signaux PPG historiques, et le fait de déterminer, sur la base de la similarité et d'une quantité d'ensembles de signaux PPG de la catégorie du premier signal PPG dans les seconds signaux PPG historiques, si le modèle cible doit être mis à jour, dans lequel
les seconds signaux PPG historiques comprennent les N ensembles de signaux PPG.

12. Procédé de mesure de la pression artérielle selon l'une quelconque des revendications 8 à 11, dans lequel la commande du premier composant de mesure (102) pour collecter N ensembles de valeurs de pression artérielle d'un utilisateur comprend :
la commande de la partie gonflable (1022) pour gonfler le coussin gonflable (1023) ; et
la commande du capteur de pression d'air (1024) pour collecter une pluralité de valeurs de pression d'air du coussin gonflable (1023), dans lequel une première valeur de pression d'air et une seconde valeur de pression d'air dans la pluralité de valeurs de pression d'air sont un ensemble de valeurs de pression artérielle de l'utilisateur, la première valeur de pression d'air est une valeur de pression d'air correspondant à un moment pendant lequel une onde oscillatoire de pression d'air dans le coussin gonflable (1023) atteint une valeur maximale, la seconde valeur de pression d'air est une valeur de pression d'air correspondant à un moment pendant lequel l'onde oscillatoire atteint la valeur maximale multipliée par a, et a est supérieur à 0 et inférieur à 1.

13. Procédé de mesure de la pression artérielle selon l'une quelconque des revendications 8 à 12, dans lequel une différence entre un moment pendant lequel le coussin gonflable (1023) commence à se gonfler et un moment de collecte du i^{ième} ensemble de signaux PPG parmi les N ensembles de signaux PPG est supérieure ou égale à un premier seuil prédéfini et est inférieure ou égale à un deuxième seuil prédéfini, et le moment pendant lequel le coussin gonflable (1023) commence à se gonfler est postérieur au moment de collecte du i^{ième} ensemble de signaux PPG, ou une différence entre un moment de collecte du i^{ième} ensemble de signaux PPG parmi les N ensembles de signaux PPG et un moment pendant lequel le gonflage du coussin gonflable (1023) est arrêté est supérieure ou égale à un troisième seuil prédéfini et est inférieure ou égale à un quatrième seuil prédéfini, et le moment pendant lequel le gonflage du coussin gonflable (1023) est arrêté est antérieur au moment de la collecte du i^{ième} ensemble de signaux PPG.

14. Support de stockage informatique, comprenant des instructions, dans lequel, lorsque les instructions sont exécutées sur un appareil de mesure de la pression artérielle, l'appareil de mesure de la pression artérielle est activé pour réaliser le procédé de mesure de la pression artérielle selon l'une quelconque des revendications 8 à 13.

15. Produit de programme informatique, dans lequel lorsque le produit de programme informatique est exécuté sur un ordinateur, l'ordinateur est activé pour réaliser le procédé de mesure de la pression artérielle selon l'une quelconque des revendications 8 à 13.
